# EUROPEAN PATENT SPECIFICATION

(11) **EP 4 400 585 B1**
(45) Date of publication and mention of the grant of the patent: **15.07.2026**
(21) Application number: 22861458.2
(22) Date of filing: 26.08.2022
(51) Int. Cl.: C12N 15/09, C07K 14/01, C12N 1/15, C12N 1/19, C12N 1/21, C12N 5/10, C12N 15/62, C12Q 1/02, C12Q 1/6897

(54) **SYSTEM FOR REGULATING PROTEIN TRANSLATION**
SYSTEM ZUR REGULIERUNG DER PROTEINTRANSLATION
SYSTÈME DE RÉGULATION DE LA TRADUCTION DE PROTÉINES

(30) Priority: 27.08.2021 JP 2021139141
(43) Date of publication of application: 17.07.2024
(73) Proprietor: The University of Osaka, Suita-shi, Osaka 565-0871 (JP)
(72) Inventor: NAKANISHI, Hideyuki, Tokyo 113-8510 (JP); ITAKA, Keiji, Tokyo 113-8510 (JP); SAITO, Hirohide, Kyoto-shi, Kyoto 606-8501 (JP)
(74) Representative: HGF
(86) International application number: PCT/JP2022/032192
(87) International publication number: WO 2023/027170

(56) References cited:
- JP-A- 2020 537 646
- HEEJAE KIM ET AL: "Bioengineering strategies to generate artificial protein complexes", BIOTECHNOLOGY AND BIOENGINEERING, JOHN WILEY, HOBOKEN, USA, vol. 112, no. 8, 19 May 2015 (2015-05-19), pages 1495 - 1505, XP071101933, ISSN: 0006-3592, DOI: 10.1002/BIT.25637
- YAO ZHONG ET AL: "Split Intein-Mediated Protein Ligation for detecting protein-protein interactions and their inhibition", vol. 11, no. 1, 15 May 2020 (2020-05-15), XP055784671, Retrieved from the Internet <URL:http://www.nature.com/articles/s41467-020-16299-1> DOI: 10.1038/s41467-020-16299-1
- NAKANISHI HIDEYUKI, SAITO HIROHIDE: "Caliciviral protein-based artificial translational activator for mammalian gene circuits with RNA-only delivery", NATURE COMMUNICATIONS, vol. 11, no. 1, XP093038940, DOI: 10.1038/s41467-020-15061-x
- GRAMESPACHER JOSEF A., BURTON ANTONY J., GUERRA LUIS F., MUIR TOM W.: "Proximity Induced Splicing Utilizing Caged Split Inteins", JOURNAL OF THE AMERICAN CHEMICAL SOCIETY, AMERICAN CHEMICAL SOCIETY, vol. 141, no. 35, 4 September 2019 (2019-09-04), pages 13708 - 13712, XP093038942, ISSN: 0002-7863, DOI: 10.1021/jacs.9b05721

## Description

### TECHNICAL FIELD

The present invention relates to a system for regulating cell-specific protein translation using a molecule in a cell as an index, and a translation regulation method employed in the same.

### BACKGROUND ART

Due to the COVID-19 pandemic, mRNA pharmaceuticals including mRNA vaccines have attracted attention. In a cell of a natural-occurring eukaryote, DNA corresponding to a genetic substance present in the nucleus of the cell is transcribed to generate mRNA, and the mRNA is translated in the cytoplasm to produce a protein. On the other hand, an mRNA pharmaceutical can produce a protein useful as a vaccine or disease treatment without using DNA present in the nucleus, but instead using externally administered artificially synthesized mRNA.

Such mRNA pharmaceuticals are novel drug discovery modalities attracting worldwide attention. These mRNA pharmaceuticals have a large number of advantages in that they are useful for expression of all types of proteins, including secretory proteins and intracellular signaling molecules, because they have no risk of canceration via insertion mutation into a genome, which differs from use of DNA, and that they produce proteins only by entering the cytoplasm, and hence are applicable, in principle, to all cells, including a non-dividing cell.

The present inventors have developed and reported an artificial translational regulator having both effects of protein translational activation and translational repression (see, for example, Non-Patent Document 1). This artificial translational regulator contains a translational activator of an imitated Cap domain, and an RNA-binding protein. The artificial translational regulator strongly binds to an mRNA containing a Cap structure, a prescribed binding motif specifically recognized by the RNA-binding protein of the artificial translational regulator, and a nucleic acid sequence encoding a desired protein, and thus can repress translation. On the other hand, the artificial translational regulator weakly binds to an mRNA not containing a regular Cap structure, and containing another prescribed binding motif specifically recognized by the RNA-binding protein of the artificial translational regulator, and a nucleic acid sequence encoding a desired protein, and thus can activate the translation. Therefore, a single artificial translational regulator can be used for repressing the translation of a given mRNA, and activating the translation of another mRNA.

### REFERENCE DOCUMENT LIST

### NON-PATENT LITERATURE

Non-Patent Document 1: H. Nakanishi and H. Saito, Nature Communications, 2020 Mar 10; 11(1): 1297.doi: 10.1038/s41467-020-15061-x
Kim et al, Biotechnology and Bioengineering, 2015 Vol. 112, No. 8 pages 1495-1505 describes bioengineering strategies to generate artificial protein complexes.

### SUMMARY OF INVENTION

### PROBLEM TO BE SOLVED BY THE INVENTION

There is, however, no report of an artificial translational regulator causing cell-specific translation. It is desired to develop a system capable of selective translational regulation of a protein of interest in accordance with the type of cell.

Any references in the description to methods of treatment which comprise use of a system of the claimed invention and which constitute a method of treatment of the human or animal body by surgery or therapy, or a diagnostic method practised on the human or animal body, are to be interpreted as references to the system for use in those methods.

### MEANS FOR SOLVING THE PROBLEMS

As a result of extensive research, the present inventors have constructed a system in which a fragment of a translational regulator is introduced into a cell to reconstruct a full length translational regulator in response to a freely selected target molecule, such as a target protein, in the cell. It has been thus found that protein translation can be regulated specifically in accordance with the type of cell with a target molecule used as an index, resulting in completion of the present invention.

Specifically, the present invention encompasses the following:
[1] A system for regulating protein translation, comprising the following mRNAs (a), (b), and (c), or DNAs encoding these mRNAs:
   (a) an mRNA that expresses a first fusion protein containing a first target-binding molecule specifically recognizing a first site of a target molecule, an N-terminal domain of a caged intein, and an N-terminal domain of a translational regulator;
   (b) an mRNA that expresses a second fusion protein containing a second target-binding molecule specifically recognizing a second site of the target molecule, a C-terminal domain of a caged intein, and a C-terminal domain of the translational regulator; and
   (c) a switch mRNA containing one or more binding motifs specifically recognizing the translational regulator, and a nucleic acid sequence encoding a protein of interest,
   in which the translational regulator contains an RNA-binding protein.
[2] The system according to [1], in which the mRNA (a) is a first mRNA containing a nucleic acid sequence encoding the first fusion protein, and the mRNA (b) is a second mRNA containing a nucleic acid sequence encoding the second fusion protein.
[3] The system for regulating protein translation according to [1], in which the mRNA (a) and the mRNA (b) form a single mRNA molecule containing, in a 5' to 3' direction, a nucleic acid sequence encoding the second fusion protein, a self-cleaving sequence encoding a self-cleaving peptide, and a nucleic acid sequence encoding the first fusion protein.
[4] The system according to any one of [1] to [3], in which the translational regulator contains the RNA-binding protein and a translational activator.
[5] The system according to any one of [1] to [4], in which the N-terminal domain of the translational regulator contains a first portion of the RNA-binding protein, and the C-terminal domain of the translational regulator contains a second portion of the RNA-binding protein, and a translational activator.
[6] The system according to any one of [1] to [5], in which the N-terminal domain of the caged intein is a caged eNpu N-intein or a mutant thereof, and the C-terminal domain of the caged intein is a caged Npu C-intein or a mutant thereof.
[7] The system according to any one of [1] to [6], in which the switch mRNA is:
   (A) an OFF switch mRNA repressed in translation by the translational regulator, and/or
   (B) an ON switch mRNA activated in translation by the translational regulator,

   the OFF switch mRNA has a cap structure or a cap analog at a 5'-end, and contains one or more binding motifs specifically recognizing the RNA-binding protein, and a nucleic acid sequence encoding a first protein of interest, and
   the ON switch mRNA does not have a 7-methylguanosine 5'-phosphate structure, and contains one or more binding motifs specifically recognizing the RNA-binding protein, and a nucleic acid sequence encoding a second protein of interest.
[8] The system according to any one of [1] to [7], in which the RNA-binding protein is MS2CP.
[9] The system according to [8], in which a binding motif for the MS2CP has a potential secondary structure represented by the following Formula (I): 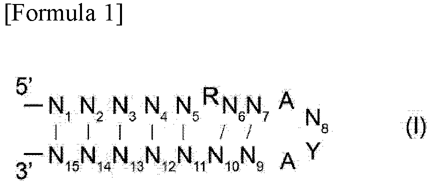 in which
   N₁ to N₁₅ each independently represent A, C, G, or U;
   R represents G or A;
   Y represents U or C; and
   N₁ and N₁₅, N₂ and N₁₄, N₃ and N₁₃, N₄ and N₁₂, N₅ and N₁₁, N₆ and N₁₀, and N₇ and N₉ each form a base pair or wobble base pair.
[10] The system according to [8] or [9], in which the OFF switch mRNA contains two or more binding motifs specifically recognizing the MS2CP, and at least two of the two or more binding motifs are linked to each other via an RNA scaffold structure.
[11] A method for regulating protein translation, comprising a step of introducing the system according to any one of [1] to [10] into a cell in vitro.
[12] The system according to any one of [1] to [10] for use in a method of regulating protein translation, the method comprising a step of introducing the system into a cell in vivo.
[13] The system for regulating protein translation according to [1],
   wherein the translational regulator is a protein containing:
   an amino acid sequence specified by SEQ ID NO: 11;
   an amino acid sequence obtained by deleting, substituting, inserting, and/or adding one to five amino acids in the amino acid sequence specified by SEQ ID NO: 11; or
   an amino acid sequence having a sequence identity of 85% or more to the amino acid sequence shown in SEQ ID NO: 11, and
   a splitting portion between an N-terminal domain and a C-terminal domain of the translational regulator is between a residue at position 45 and a residue at position 46, or a portion away from the splitting portion toward an N-terminal side or a C-terminal side by five amino acid residues or less.
[14] The system for regulating protein translation according to [1], wherein the translational regulator contains an amino acid sequence specified by SEQ ID NO: 11, the N-terminal domain of the translational regulator contains residues at positions 1 to 45 of SEQ ID NO: 11, and the C-terminal domain of the translational regulator contains residues at positions 46 to 116 of SEQ ID NO: 11.

### ADVANTAGEOUS EFFECTS OF INVENTION

According to the present invention, a translational regulator can be reconstituted specifically to a cell type containing a desired target molecule. Then, the translational regulator is caused to act on a switch mRNA encoding a protein of interest, and thus, translational repression and/or translational activation of the protein of interest can be conducted. In addition, when a target-binding molecule binding to a target molecule is exchanged, the translational regulator can be reconstituted specifically by a freely selected target molecule, and therefore, cell specific translational repression and/or translational activation of a protein of interest can be realized in a freely selected cell.

### BRIEF DESCRIPTION OF DRAWINGS

FIG. 1A, 1B and 1C are conceptual diagrams respectively illustrating a state in which first and second mRNAs are introduced into a cell and the first and second mRNAs are translated, a state in which a first fusion protein and a second fusion protein both bind to a target protein, and a reconstructed full length translational regulator (left), and a complex (right) derived from a caged intein detached due to the action of the caged intein, a target-binding molecule, and a target protein.
FIG. 2 is a diagram schematically illustrating introduction of first mRNA, second mRNA, OFF switch mRNA, and ON switch mRNA into a cell.
FIG. 3 is a diagram schematically illustrating that a reconstituted full length translational regulator regulates both the ON switch mRNA (lower left) and the OFF switch mRNA ((lower right).
FIG. 4A is a diagram illustrating a plasmid DNA construct designed in preliminary experiment using Npu DnaE intein, and FIG. 4B is a graph illustrating results of translational repression obtained by only the N-terminal, or by only the C-terminal of a translational repressor, and by a reconstituted translational repressor in each split site.
FIG. 5A is a diagram illustrating a plasmid DNA construct encoding first and second mRNAs according to Example 1 of the present invention (left), a DNA construct encoding an OFF switch mRNA (upper right), a DNA construct encoding Nluc used as a control for measuring the expression level of a protein of interest (middle right), and an mRNA encoding a target protein eDHFR, and FIG. 5B is a graph illustrating results of translational repression obtained by introducing these constructs and mRNAs.
FIG. 6A is a diagram illustrating, in the downward direction, a first mRNA according to Example 2 of the present invention, a second mRNA, an mRNA encoding a target protein eDHFR, an mRNA encoding EGFP, that is, a negative control of the target protein, an OFF switch mRNA using Luc2 as a protein of interest, an ON switch mRNA using Luc2 as a protein of interest, and an mRNA encoding Nluc used as a control for measuring the expression level of the protein of interest.
FIG. 6B is a graph illustrating results of translational repression of the OFF switch mRNA obtained by introducing the respective mRNAs into a cell.
FIG. 6C is a graph illustrating results of translational activation of the ON switch mRNA obtained by introducing the respective mRNAs into a cell.
FIG. 7A is a diagram illustrating, in the downward direction, a first mRNA according to Example 3 of the present invention, a second mRNA, an mRNA encoding a target protein EGFP, an mRNA encoding eDHFR, that is, a negative control of the target protein, an OFF switch mRNA using Luc2 as a protein of interest, an ON switch mRNA using Luc2 as a protein of interest and an mRNA encoding Nluc used as a control for measuring the expression level of the protein of interest.
FIG. 7B is a graph illustrating results of translational repression of the OFF switch mRNA obtained by introducing the respective mRNAs into a cell.
FIG. 7C is a graph illustrating results of translational activation of the ON switch mRNA obtained by introducing the respective mRNAs into a cell.

### MODE FOR CARRYING OUT THE INVENTION

Preferred embodiments of the present invention will now be described. It is to be noted that the present invention is not limited to the following embodiments.

The present invention relates to system and method for regulating protein translation. The system of the present invention contains the following mRNAs (a), (b), and (c), or DNAs encoding these mRNAs:
(a) an mRNA that expresses a first fusion protein containing a first target-binding molecule specifically recognizing a first site of a target molecule, an N-terminal domain of a caged intein, and an N-terminal domain of a translational regulator;
(b) an mRNA that expresses a second fusion protein containing a second target-binding molecule specifically recognizing a second site of the target molecule, a C-terminal domain of the caged intein, and a C-terminal domain of the translational regulator; and
(c) a switch mRNA containing one or more binding motifs specifically recognizing the translational regulator, and a nucleic acid sequence encoding a protein of interest.

The translational regulator contains an RNA-binding protein.

According to a first embodiment of the present invention, in the system of the present invention, the mRNA (a) is a first mRNA containing a nucleic acid sequence encoding a first fusion protein that contains a first target-binding molecule specifically recognizing a first site of a target molecule, an N-terminal domain of a caged intein, and an N-terminal domain of a translational regulator, and the mRNA (b) is a second mRNA containing a nucleic acid sequence encoding a second fusion protein that contains a second target-binding molecule specifically recognizing a second site of the target molecule, a C-terminal domain of the caged intein, and a C-terminal domain of the translational regulator. Specifically, in the first embodiment, the mRNA containing the nucleic acid sequence encoding the first fusion protein and the mRNA containing the nucleic acid sequence encoding the second fusion protein are contained as different molecules.

Now, system and method for regulating protein translation according to the first embodiment will be described. The method according to the present invention includes a step of introducing the mRNAs (a), (b), and (c), or DNAs encoding these mRNAs into a cell.

The present embodiment relates to system and method for regulating protein translation, and more particularly, relates to system and method for regulating translation of a protein of interest cell-specifically with a target molecule used as an index. To regulate translation of a protein of interest cell-specifically with a target molecule used as an index refers to "to make translation of the protein of interest encoded by the switch mRNA (c) regulatable in a cell in which the target molecule is present". More specifically, it means that the translation amount of the protein of interest encoded by the switch mRNA is increased (that is, the translation of the protein of interest is activated), or the translation amount of the protein of interest is reduced (that is, the translation of the protein of interest is repressed) in the presence of a reconstituted translational regulator as compared with that obtained when the translational regulator is absent or in a split state. Herein, a cell type for causing specific translational regulation is referred to also as a target cell. In addition, the other cell types are also referred to as non-target cells.

Herein, only in a cell in which the target molecule is present, is the translational regulator reconstituted based on the first fusion protein expressed by the mRNA (a) and the second fusion protein expressed by the mRNA (b), and the reconstituted translational regulator makes the translation of the switch mRNA (c) regulatable.

A target molecule refers to a molecule present in a target cell at the time of conducting the method according to the present invention, and in one embodiment, can refer to a molecule present in the cytoplasm or nucleus of the target cell. The target molecule is not particularly limited as long as it has a first binding site and a second binding site specifically recognizable by another molecule. The first binding site and the second binding site of the target molecule are, however, preferably different sites. In addition, the target molecule is preferably non-toxic to a cell. Specific examples of the target molecule include a protein, a peptide, a non-peptide compound, a synthesized low molecular weight compound, a natural compound, and fragments thereof. The target molecule can be appropriately selected in accordance with the purpose of the translational regulation, and for example, when the expression of the protein of interest is desired to be regulated in accordance with the intracellular state, the target molecule may be a protein or a peptide as long as it is a molecule specific to the intracellular state to be detected. Alternatively, when the regulation is desired to be conducted by externally introducing the target molecule, a low molecular weight compound easily permeating the cell membrane can be selected. Alternatively, a metabolite generated as a result of a natural or artificial reaction within a cell can be used as the target molecule, and the metabolite in such a case may be a low molecular weight compound or the like.

Hereinafter, a case in which the target molecule is a protein or a fragment thereof in the present invention will be mainly described. Hereinafter, a target molecule containing a protein or a fragment thereof is referred to also as a "target protein". In the method according to the present invention, for purposes of specifically repressing or activating expression of a protein of interest in a target cell, the target cell is selected and determined, and the mRNAs (a) and (b) can be designed based on the target protein. As the target protein, for example, a protein that is largely different in expression level or abundance between a target cell and a non-target cell is preferably selected. As a target molecule that is not a target protein, a molecule that is largely different in abundance between a target cell and a non-target cell is preferably selected.

An example of the target protein includes a protein naturally occurring in a specific cell. Examples of the protein naturally occurring in a specific cell include, but are not limited to, a protein expressed in accordance with the stage of differentiation of the cell, and a protein expressed in accordance with a disease state of the cell. When the translational regulator is reconstituted in accordance with the presence of such a target protein, the translation of the protein of interest encoded by the switch mRNA can be regulated in a cell at the differentiation stage or a cell in a disease state characterized by the target protein. Another example of the target protein includes a protein generated due to a substance externally introduced into the cell. Examples of the protein generated due to a substance externally introduced into the cell include, but are not limited to, a protein that can be generated in the cell due to a gene repaired by genome editing, a protein that can be generated in the cell by mRNA vaccine inoculation or an mRNA pharmaceutical, and a protein that can be generated in the cell by a plasmid DNA or a viral vector. In addition, examples of a target molecule that is not a target protein include a molecule produced in the cell in a specific disease state, and a molecule artificially produced in the cell, and the type is not limited. When the translational regulator is reconstituted using, as an index, the presence of such a target molecule, particularly a target protein, the translation of the protein of interest encoded by the switch mRNA can be regulated in the cell in which the target molecule is present.

The term "cell" used herein is not especially limited, and may be a freely selected cell. The cell may be a single cell, or may be a "cell population" that is a gathering of two or more cells. The "cell population" is not theoretically limited in the upper limit of the number of cells, and refers to a group of a freely selected number of cells. For example, the "cell population" may be a cell population that contains a plurality of different types of cells, and can contain a target cell and a non-target cell.

The "cell" may be a cell collected from a unicellular organism or a multicellular organism, or may be a cell (including a cell line) artificially obtained. For example, yeasts, insect cells, animal cells and the like are used, among which animal cells are preferred. Examples of the animal cells include cells derived from mammals (such as a mouse, a rat, a hamster, a guinea pig, a dog, a monkey, an orangutan, a chimpanzee, and a human). A cell derived from a mammal may be a cell line of, for example, monkey COS-7 cell, monkey Vero cell, Chinese hamster ovarian (CHO) cell, dhfr gene-deleted CHO cell, mouse L cell, mouse AtT-20 cell, mouse myeloma cell, rat GH3 cell, human embryo kidney-derived cell (such as HEK293 cell), human hepatoma-derived cell (such as HepG2), or human FL cell, and a primary cultured cell prepared from a tissue of a human or another mammal is used. In addition, a zebrafish embryo, a Xenopus oocyte or the like can be used.

The degree of differentiation of the cell, the age of the animal from which the cell is collected, and the like are not particularly limited, and the cell may be any one of a stem cell (A), a progenitor cell (B), a terminally differentiated somatic cell (C), and another cell (D). Examples of the stem cell (A) include, but are not limited to, an embryonic stem (ES) cell, a non-human nuclear transfer cloned embryo-derived embryonic stem (ntES) cell, a spermatogonial stem cell ("GS cell"), an embryonic germ cell ("EG cell"), and an artificial pluripotent stem (iPS) cell. Examples of the progenitor cell (B) include tissue stem cells (somatic stem cells) such as a neural stem cell, a hematopoietic stem cell, a mesenchymal stem cell, and a dental pulp stem cell. Examples of the somatic cell (C) include keratinized epithelial cells (such as a keratinized epidermal cell), mucosal epithelial cells (such as an epithelial cell of the tongue surface layer), exocrine epithelial cells (such as a mammary gland cell), hormone secreting cells (such as an adrenomedullary cell), cells for metabolism/storage (such as a liver cell), inner luminal epithelial cells constituting a boundary surface (such as a type I alveolar cell), inner luminal epithelial cells of the inner chain tube (such as a vascular endothelial cell), cells having cilia with transport ability (such as a respiratory tract epithelial cell), cells for extracellular matrix secretion (such as a fibroblast), contractile cells (such as a smooth muscle cell), cells of blood and immune system (such as a T lymphocyte), cells regarding senses (such as a rod cell), nerve cells and glial cells in the central/peripheral nervous system (such as an astroglial cell), pigment cells (such as a retinal pigment epithelial cell), and the progenitor cells thereof (tissue progenitor cells). Examples of another cell (D) include cells obtained through differentiation induction, and include progenitor cells and somatic cells differentiation-induced from pluripotent stem cells. Alternatively, the cell may be a cell that has been differentiated into a desired cell directly from a somatic cell or a progenitor cell without through an undifferentiated state, and induced by what is called a "direct conversion (which is also referred to as direct reprogramming or trans-differentiation)".

In the first embodiment, the first mRNA (a) and the second mRNA (b) are mRNAs respectively expressing the first and second fusion proteins. The first fusion protein contains the N-terminal domain of the translational regulator, and the second fusion protein contains the C-terminal domain of the translational regulator. Thus, the first and second fusion proteins associate with each other when the target protein is present in the cell, and reconstitute the full length translational regulator in the cell.

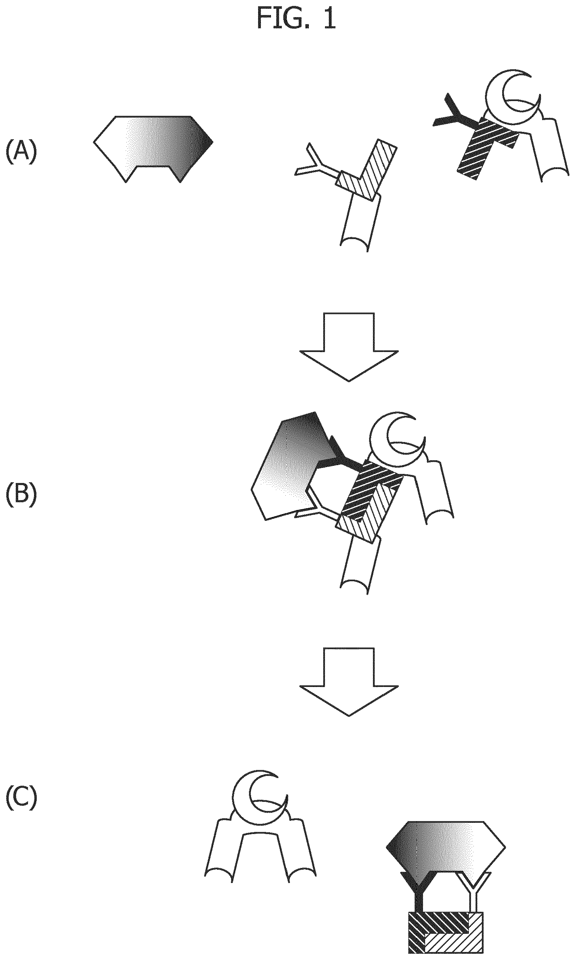

Referring to FIG. 1, the action of the first mRNA (a) and the second mRNA (b) introduced into the cell will be described. FIG. 1A is a conceptual diagram illustrating a state in which the first and second mRNAs are introduced into a cell, and the first and second mRNAs are translated. FIG. 1A schematically illustrates the target protein (left), the first fusion protein (middle), and the second fusion protein (right). The first fusion protein is a substance generated when the first mRNA is translated in the cell, and contains the N-terminal domain of the translational regulator, the N-terminal domain of a caged intein, and the first target-binding molecule bound in the stated order from the N-terminal. The second fusion protein is a substance generated when the second mRNA is translated in the cell, and contains the second target-binding molecule, the C-terminal domain of the caged intein, and the C-terminal domain of the translational regulator bound in the stated order from the N-terminal.

When the target protein is present in the cell, the first target-binding molecule of the first fusion protein specifically recognizes and binds to a first site of the target protein, and the second target-binding molecule of the second fusion protein specifically recognizes and binds to a second site of the target protein. FIG. 1B illustrates a state in which both the first fusion protein and the second fusion protein are binding to the target protein. Thus, the N-terminal domain of the caged intein and the C-terminal domain of the caged intein bind to each other. Subsequently, due to the action of the caged intein, the N-terminal domain and the C-terminal domain of the translational regulator bind to each other to reconstitute the full length translational regulator. FIG. 1C illustrates the thus reconstituted full length translational regulator (left), and a complex (right) derived from the caged intein detached due to the action of the caged intein, the target-binding molecule, and the target protein.

The design of the first and second fusion proteins will now be described. In the design of the first and second fusion proteins, the full length translational regulator to be reconstituted is designed. The translational regulator is an artificial translational regulator in general, and contains at least an RNA-binding protein. More specifically, a translational regulator to be used for translational repression contains or consists of an RNA-binding protein. A translational regulator to be used for translational activation contains an RNA-binding protein and a translational activator. Herein, the term "full length translational regulator" refers to a translational regulator that is obtained by reconstitution of the N-terminal domain and the C-terminal domain, and has translational regulation function for a switch mRNA.

An RNA-binding protein refers to a protein that specifically recognizes a specific RNA sequence to form an RNA-protein complex. Herein, such a specific RNA sequence that an RNA-binding protein specifically recognizes and binds to is referred to as a "binding motif" or "protein-binding motif". Any RNA-binding protein can be used in the present invention without limitation as long as it is a protein capable of binding to a binding motif. Specific examples of the RNA-binding protein include MS2CP, L7Ae, LIN28A, PP7CP, dCas13, modified products of these (for example, V29I mutant, L77P mutant, W82R mutant, C101R mutant, dlFG mutant and the like of MS2CP), and fragments thereof having RNA-binding ability. The translational activation and/or repression using MS2CP, L7Ae, and LIN28A has already been verified. The RNA-binding protein is preferably MS2CP or a modified product thereof. In one embodiment of the present invention, the RNA-binding protein can be a protein containing an amino acid sequence shown in any of SEQ ID NOS: 5, 7 and 9 (with nucleotide sequences encoding the proteins of these sequences respectively shown in SEQ ID NOS: 4, 6 and 8), or a protein containing an amino acid sequence obtained by deleting, substituting, inserting, and/or adding one or several (for example, 2, 3, 4, or 5) amino acids in the amino acid sequence shown in any of SEQ ID NOS: 5, 7, and 9, or an amino acid sequence having a sequence identity of 85% or more (for example, 90%, 95%, 96%, 97%, 98%, 99%, or more) to the amino acid sequence shown in any of SEQ ID NOS: 5, 7, and 9. Herein, the RNA-binding protein encompasses a wild-type RNA binding protein, a modified product thereof, and a fragment thereof.

The sequence identity of an amino acid sequence can be calculated under the following conditions (expectancy = 10; gap allowed; matrix = BLOSUM62; filtering = OFF) using homology calculation algorithm, NCBI BLAST (National Center for Biotechnology Information Basic Local Alignment Search Tool) (https://blast.ncbi.nlm.nih.gov/Blast.cgi).

The translational activator is not particularly limited as long as it can activate translation by recruiting a translation initiation factor. In one embodiment of the present invention, the translational activator encompasses a VPg (viral protein genome-linked) protein. Hereinafter, description will be given by exemplifying a VPg as the translational activator, and when a translational activator different from a VPg is used, the "VPg" shall be read as the different translational activator. The translational regulation system of the present invention can activate or repress protein translation owing to the configuration described so far.

The VPg protein is not particularly limited as long as it has protein translational activation function, and examples include VPg proteins derived from viruses belonging to the genus *Vesivirus,* the genus *Lagovirus,* and the genus *Potyvirus,* modified products of these VPg proteins, and fragments thereof having the translational activation function. Although not limiting, examples of the viruses belonging to the genus *Vesivirus* include feline calicivirus (NCBI Accession No: NP_783307.1), canine herpesvirus (NCBI Accession No: NP_786908.1), and vesicular exanthema virus (NCBI Accession No: NP_786894.1), an example of viruses belonging to the genus *Lagovirus* includes rabbit hemorrhagic disease virus VPg (NCBI Accession No: NP_740330.1), and examples of viruses belonging to the genus *Potyvirus* include Lupinus mosaic virus, Bean yellow mosaic virus, and Papaya leaf distortion mosaic virus. Viruses belonging to the genus *Vesivirus* are preferred, and feline calicivirus is particularly preferred. In one embodiment of the present invention, the VPg protein can be a protein containing an amino acid sequence shown in SEQ ID NO: 3 (with a nucleotide sequence encoding the protein of this sequence shown in SEQ ID NO: 2), or a protein containing an amino acid sequence obtained by deleting, substituting, inserting, and/or adding one or several (for example, 2, 3, 4, or 5) amino acids in the amino acid sequence shown in SEQ ID NO: 3, or an amino acid sequence having a sequence identity of 85% or more (for example, 90%, 95%, 96%, 97%, 98%, 99%, or more) to the amino acid sequence shown in SEQ ID NO: 3. Herein, the "VPg protein" encompasses a VPg protein, a modified product of a VPg protein, and a fragment thereof.

In one embodiment, a translational regulator having functions of both translational repression and translational activation may be a fusion protein containing an RNA-binding protein and a VPg protein. Alternatively, the translational regulator may be obtained by fusing a protein-binding domain, such as SH3 domain, PDZ domain, GK domain, or GB domain, and a binding partner thereof respectively with an RNA-binding protein and a VPg protein.

When the translational regulator is a fusion protein, the RNA-binding protein and the VPg protein may be present respectively on the N-terminal side and the C-terminal side, the RNA-binding protein and the VPg protein may be present respectively on the C-terminal side and the N-terminal side, and a linker may be present therebetween.

In a preferred embodiment of the present invention, the translational regulator having the functions of both translational repression and translational activation may be a fusion protein of an RNA-binding protein and a VPg protein, and may be a Caliciviral VPg-based Translational activator (CaVT) disclosed in Non-Patent Document 1. This translational regulator can be a protein containing an amino acid sequence shown in SEQ ID NO: 11 (with a nucleotide sequence encoding the protein of this sequence shown in SEQ ID NO: 10), or a protein containing an amino acid sequence obtained by deleting, substituting, inserting, and/or adding one or several (for example, 2, 3, 4, or 5) amino acids in the amino acid sequence shown in SEQ ID NO: 11, or an amino acid sequence having a sequence identity of 85% or more (for example, 90%, 95%, 96%, 97%, 98%, 99%, or more) to the amino acid sequence shown in SEQ ID NO: 11.

In designing the first and second fusion proteins, the translational regulator is split into the N-terminal domain and the C-terminal domain. The position for splitting the translational regulator into the N-terminal domain and the C-terminal domain is not especially limited as long as the following conditions are satisfied. Specifically, the conditions are such that each of the domains before reconstitution does not have a function such as translational repression or translational activation; that the translational regulator is reconstituted by a caged intein; and that the function of the translational regulator is maintained after the reconstitution. For example, the translational regulator is split into the N-terminal domain and the C-terminal domain preferably in an aspect in which the RNA-binding protein, that is, the essential component thereof, is split. In addition, the translational regulator can be split into the N-terminal domain and the C-terminal domain in a loop portion constituting neither α helix nor β-sheet in analysis of the three-dimensional structure of the RNA-binding protein.

In a translational regulator having only the translational repression function, the N-terminal domain contains an N-terminal fragment of the RNA-binding protein, and the C-terminal domain contains a C-terminal fragment of the RNA-binding protein.

In the translational regulator having the functions of both translational repression and translational activation, the N-terminal domain contains an N-terminal fragment of the RNA-binding protein, and the C-terminal domain contains a C-terminal fragment of the RNA-binding protein and a translational activator in one embodiment. In another embodiment, the N-terminal domain of the translational regulator contains a translational activator, and an N-terminal fragment of the RNA-binding protein, and the C-terminal domain of the translational regulator contains a C-terminal fragment of the RNA-binding protein. For example, when the translational regulator is CaVT, the splitting portion may be, for example, between tyrosine at position 45 and cysteine at position 46. Alternatively, the splitting portion may be changed from this splitting portion toward the N-terminal side or the C-terminal side by 5 amino acid residues or less, and in this case, a site constituting neither α helix nor β-sheet is preferably selected. An appropriate splitting portion can be confirmed though preliminary experiment or simulation performed by those skilled in the art.

The caged intein is a protein that causes, differently from a usual intein, reconstitution only when there is interaction via another domain, and is defined as an intein with which a cage structure is fused for preventing reconstitution independent of interaction via another domain. In addition, as the cage structure for N-intein, a protein or peptide having a C-intein-like sequence can be used, and as the cage structure for C-intein, a protein or peptide having an N-intein-like sequence can be used. In the present invention, another domain is the first and second target-binding molecules. As the caged intein, a freely selected one can be used. For example, a caged eNpu N-intein (SEQ ID NO: 14) that is an

N-terminal domain of a caged intein, and a caged Npu C-intein (SEQ ID NO: 15) that is a C-terminal domain thereof disclosed in Gramespacher J. A. et al., J. Am. Chem. Soc. 2019, 141, 35, 13708-13712 can be used, which is not limiting. The respective sequences are shown in Table 1. In the Table, characters in bold type show an N-intein-derived sequence (in SEQ ID NO: 14) or a C-intein-derived sequence (in SEQ ID NO: 15), underlined characters show a linker sequence, and boxed letters show a C-intein-like sequence (in SEQ ID NO: 14), and an N-intein-like sequence (in SEQ ID NO: 15).

**Table 1**

| |
|---|
| Caged eNpu N-Intein SEQ ID NO: 14 |
| CLSYETEILTVEYGLLPIGKIVEKRIECTVYSVDNNGNIYTQPVAQWHDRGKQ KVFEYCLEDGSLIRATKDHKFMTVDGQMLPIKEIFRRKLDLMRVDNLPNGSGG ENLYFQGENLYFQGGSGGIEIATEKYLGEQNVVYDIGVERDHNFALKNGGYFQGIEI ATEKYLGEQNVVYDIGVERDHNFALKNG |
| Caged Npu C- Intein SEQ ID NO: 15 |
| ;GEQEVFEYCLEDGSLIRATKDHKFMTVDGQMLPIDEIFERELDLMRVDNLPN;GSG GENLYFQGENLYFQGGSGGIKIATRKYLGKQNVYDIGVERDHNFALKNGFIASN |

In SEQ ID NO: 14, an original sequence derived from an N-intein corresponds to residues at positions 1 to 102. A sequence consisting of 30 residues at positions 125 to 154 and a sequence consisting of 30 residues at positions 160 to 189 are the same, and are C-intein-like sequences. A sequence consisting of 22 residues at positions 103 to 124 is a linker sequence of the N-intein-like sequence and the C-intein-like sequence. A sequence consisting of 5 residues at positions 155 to 159 is a linker sequence between the C-intein-like sequences. The caged eNpu N-intein shown in SEQ ID NO: 14 is a sequence consisting of 2 repeats of the C-intein-like sequence, and a sequence consisting of 1 repeat of the C-intein-like sequence, a sequence consisting 3 or more repeats thereof, and a sequence in which the types of amino acids in the linker sequence are changed can be also used in the same manner as the caged eNpu N-intein. Similarly, a sequence consisting of 52 residues at positions 1 to 52 in the SEQ ID NO: 15 is an N-intein-like sequence, a sequence consisting of 22 residues at positions 53 to 74 is a linker sequence of the N-intein-like sequence and the C-intein-like sequence, and a sequence consisting of 35 residues at positions 75 to 109 is a C-intein-derived sequence. A sequence consisting of 2 repeats of the N-intein-like sequence in the caged Npu C-intein, a sequence consisting of 3 or more repeats thereof, and a sequence in which the types of amino acids in the linker sequence are changed can be also used in the same manner as the caged Npu C-intein.

As long as the activity of the caged intein is not impaired, a mutant of the caged eNpu N-intein and a mutant of the caged Npu C-intein can be used. Examples of such mutants include, but are not limited to, a mutant obtained by conservative substitution of the amino acid sequence of SEQ ID NO: 14 or 15, and a mutant containing an amino acid sequence obtained by deleting, substituting, inserting, and/or adding one or several (for example, 2, 3, 4, or 5) amino acids in the amino acid sequence of SEQ ID NO: 14 or 15, or an amino acid sequence having a sequence identity of 85% or more (for example, 90%, 95%, 96%, 97%, 98%, 99%, or more) to the amino acid sequence shown in SEQ ID NO: 14 or 15.

The first and second target-binding molecules are molecules respectively specifically recognizing the first and second sites of a target molecule, particularly a target protein. The first and second target-binding molecules can be selected or designed so as to be compatible with the target protein. The target-binding molecule may be any molecule having binding ability to the target molecule, and when the target molecule is an antigen, examples include an antibody, a peptide aptamer, a fibronectin-derived artificial peptide, and DARPin. Also, when the target molecule is a molecule excluding a protein, a molecule specifically binding to the target molecule can be appropriately determined. For example, for a low molecular weight compound, a target-binding molecule utilizing interaction such as bonding between FKBP (FK506-binding protein) and FRB (FKBP-rapamycin binding protein) via rapamycin can be used.

The derivation of the antibody used in the present invention is not especially limited, and the antibody is preferably derived from a mammal. The antibody used in the present invention may be a full-body antibody, or a fragment of an antibody (such as a nanobody antibody, Fab, or F(ab')₂), or a modified product thereof (such as scFv), and herein, the term "antibody" is used in the sense encompassing these. The peptide aptamer used in the present invention may be a peptide having modification in the amino acid residue or both end portions. The peptide aptamer can be selected by a method known to those skilled in the art. Although not limiting, for example, the peptide aptamer can be selected by a yeast, two-hybrid method. Other molecules can be appropriately produced based on methods known per se. The target-binding molecule may be an artificial molecule obtained by modifying a protein of a different type from the antibody to provide antigen-binding ability similar to that of the antibody, and examples include, but are not limited to, a monobody, an affibody, and an anticalin.

For example, when two or more target-binding molecules respectively specifically recognizing different portions of the target protein are known from databases or literature, the first and second target-binding molecules can be selected from these known target-binding molecules. In the target protein, the first site and the second site are different portions, and it is preferable that the first site and the second site be proximal to each other, as long as their bonds to the target-binding molecule are not mutually impaired. This is because reconstitution of the caged intein is caused by interaction between the first site of the target protein and the first target-binding molecule and interaction between the second site of the target protein and the second target-binding molecule.

When a target-binding molecule specifically recognizing the target protein is unknown, or when only one such target-binding molecule is known, the target protein or a fragment thereof is used as an antigen to produce an antibody by immunizing an animal of the camelid family such as an alpaca, and a sequence of an antigen recognition site of the antibody is obtained. In this manner, two or more target-binding molecules such as nanobodies respectively specifically recognizing different sites of the target protein can be designed.

In this manner, the full-length translational regulator, the N-terminal domain of the translational regulator, the N-terminal domain of the caged intein, and the first target-binding molecule can be selected or designed. Then the first fusion protein can be designed and the structure of the first fusion protein can be determined. Similarly, the second target-binding molecule, the C-terminal domain of the caged intein, and the C-terminal domain of the translational regulator can be selected or designed. Then the second fusion protein can be designed and the structure of the second fusion protein can be determined. In the first and second fusion proteins, the respective domains may be directly bound, or may be bound via a linker. The length of the linker is not especially limited, and may be, for example, about 2 to 30 amino acids, or about 2 to 15 amino acids.

When the structures of the first and second fusion proteins are determined, the structures of the first and second mRNAs expressing these can be determined. The first mRNA may have a structure in which [5' UTR containing a cap structure or cap analog at the 5'-end], [open reading fame containing a nucleic acid sequence encoding the first fusion protein], and [3' UTR containing a poly-A] are linked in the stated order in the 5'-end to 3'-end direction.

The 5' UTR of the first mRNA contains a cap structure or a cap analog at the 5'-end. The cap structure may be 7-methylguanosine 5'-phosphate. The cap analog is a modification structure recognized by eIF4E, that is, a translation initiation factor, similarly to the cap structure, and examples include, but are not limited to, Anti-Reverse Cap Analog (ARCA) of Ambion, m7G(5')ppp(5')G RNA Cap Structure Analog of New England Biolabs, and Clean Cap of TriLink. The cap analog may be another modification structure recognized by a translation initiation factor. On the 3' side of the cap structure or the cap analog and on the 5' side of the start codon, an optional nucleic acid sequence of, for example, about 0 to 500 bases, preferably about 20 to 200 bases may be contained. Such an optional nucleic acid sequence is preferably a nucleic acid sequence not forming a secondary structure and not interacting specifically with the second mRNA. In addition, it is preferable that AUG corresponding to the start codon is not present in the 5' UTR.

The open reading frame corresponding to the coding region of the first mRNA contains the start codon, a nucleic acid sequence encoding the first fusion protein, and the stop codon. The nucleic acid sequence encoding the first fusion protein contains, in the direction from the 3' side of the start codon AUG to the 5' side of the stop codon, a nucleic acid sequence encoding the N-terminal domain of the translational regulator, a nucleic acid sequence encoding the N-terminal domain of the caged intein, and a nucleic acid sequence encoding the first target-binding molecule in the stated order. The nucleic acid sequences encoding the respective domains can be determined based on the structure of the first fusion protein precedently designed.

The 3' UTR of the first mRNA contains a poly-A tail. The poly-A tail may be a sequence consisting of about 50 to 250 adenine bases bound one to another. The about 50 to 250 adenine bases are, however, not necessarily continuously bound, and other nucleic acid bases may be contained between the adenine bases as long as the stability of the mRNA can be retained.

The second mRNA may have a structure in which [5' UTR containing a cap structure or a cap analog at the 5'-end], [open reading frame containing a nucleic acid sequence encoding the second fusion protein], and [3' UTR containing a poly-A] linked in the stated order in the 5'-end to 3'-end direction. The configurations of the [5' UTR containing a cap structure or a cap analog at the 5'-end] and the [3' UTR containing a poly-A] can be the same as those of the first mRNA. The nucleobase sequences and the number of nucleic acid bases of the 5' UTR and the 3' UTR may be the same in or different between the first mRNA and the second mRNA, and are preferably the same.

The open reading frame corresponding to the coding region of the second mRNA contains the start codon, a nucleic acid sequence encoding the second fusion protein, and the stop codon. The nucleic acid sequence encoding the second fusion protein contains, in the direction from the 3' side of the start codon AUG to the 5' side of the stop codon, a nucleic acid sequence encoding the second target-binding molecule, a nucleic acid sequence encoding the C-terminal domain of the caged intein, and a nucleic acid sequence encoding the C-terminal domain of the translational regulator in the started order. The nucleic acid sequences encoding the respective domains can be determined based on the structure of the second fusion protein precedently designed.

### (c) Switch mRNA

A switch mRNA is an mRNA in which translation is regulated by a translational regulator. The switch mRNA is divided into an ON switch mRNA in which translation is activated by a translational regulator, and an OFF switch mRNA in which translation is repressed. Both the ON switch mRNA and the OFF switch mRNA contain one or more binding motifs specifically recognizing the RNA-binding protein constituting the translational regulator, and a nucleic acid sequence encoding a protein of interest. A binding motif refers to a nucleic acid sequence specifically recognizing a specific RNA-binding protein to form an RNA-protein complex. Next, the design and the structures of the ON switch mRNA and the OFF switch mRNA will be described.

### (A) OFF Switch mRNA

An OFF switch mRNA has a cap structure or a cap analog at the 5'-end, and contains one or more binding motifs specifically recognizing an RNA-binding protein constituting a translational regulator, and a nucleic acid sequence encoding a first protein of interest.

The OFF switch mRNA of the present invention typically contains one or more binding motifs in the 5' UTR, and the binding motif is stabilized by an RNA scaffold structure. In one embodiment of the present invention, the OFF switch mRNA of the present invention preferably has binding motifs for two or more RNA binding proteins, and at least two of the binding motifs are linked to each other via an RNA scaffold structure. Herein, the term "RNA scaffold structure" means a structure that is linked to one or more binding motifs to stabilize the motifs, such a structure is not particularly limited, and an example includes a structure having one or more stem regions by base paring. A base pair of the stem region can be designed with desired stability (with the stability probably improved usually in accordance with the number of base pairs forming the stem region). In addition, when two or more binding motifs are linked, at least one may have a scaffold structure, and from the viewpoint of stability, the scaffold structure is linked preferably to all the binding motifs. In addition, the two or more motifs may be linked to each other via another structure (such as a loop structure) with or without including a stem region. Herein, to stabilize a binding motif means that the structural fluctuation of the RNA is reduced to increase a probability that the RNA has a potential secondary structure described below as compared with that in a case in which no scaffold structure is used.

The binding motif for the RNA-binding protein constituting the OFF switch mRNA of the present invention is not especially limited as long as it binds to the protein. Through the bond between the RNA-binding protein and the RNA via the binding motif, the translation of the protein of interest is repressed. For example, when the RNA-binding protein is MS2CP, the binding motif can be appropriately designed, for example, by considering a known literature (such as Grahn E et al., RNA. 7(11): 1616-1627 (2001)). A specific example includes an mRNA in which at least one of binding motifs has a potential secondary structure represented by the following Formula (I) (in which nucleotide sequence is shown in SEQ ID NO: 1): 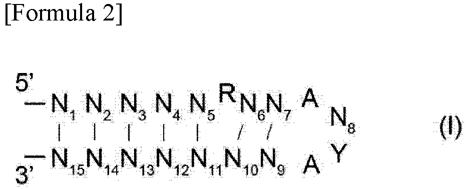 wherein
N₁ to N₁₅ each independently represent A, C, G, or U;
R represents G or A;
Y represents U or C; and
N₁ and N₁₅, N₂ and N₁₄, N₃ and N₁₃, N₄ and N₁₂, N₅ and N₁₁, N₆ and N₁₀, and N₇ and N₉ each form a base pair or wobble base pair.
In one embodiment, N₁ is A, N₂ is G or C (preferably G), N₃ is G, N₄ is U, N₅ is G, N₆ is G, N₇ is G, N₈ is U, N₉ is C, N₁₀ is C, N₁₁ is C, N₁₂ is A, N₁₃ is U, N₁₄ is C or G (preferably C), N₁₅ is U, R is A, and Y is C in the structure.

As a structure obtained by further stabilizing the Formula (I), a structure represented by the following Formula (II) can be also preferably used as the binding motif: 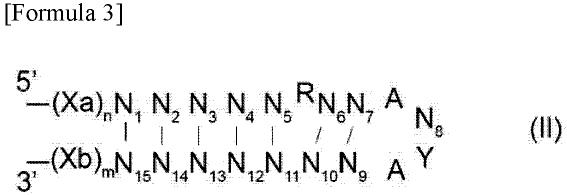
wherein N₁ to N₁₅, R, and Y are the same as defined above;
Xa and Xb each independently represent A, G, C, or U;
n and m each independently represent an integer of 0 or more; and
Xa constitutes, together with Xb, a stem structure optionally having a bulge loop.
In the Formula (II), n and m each independently typically represent an integer of 1000 or less, and preferably 100 or less (for example, 50, 20, or 10).

Herein, the potential secondary structure such as the structure represented by the Formula (I) or (II) is sometimes referred to as the "binding motif of the RNA-binding protein" (or simply abbreviated as "binding motif"), and when the RNA-binding protein is MS2CP, it is referred to also as "MS2CP-binding motif". A potential secondary structure refers to a secondary structure stably present under physiological conditions, and it can be determined, by using a structure prediction program (ParasoR) described in Example (Kawaguchi, R. & Kiryu, H. Parallel computation of genome-scale RNA secondary structure to detect structural constraints on human genome, BMC Bioinformatics 17, 203 (2016)) or the like, whether or not an RNA has a potential secondary structure.

In the Formula (II), when n and m are different integers, Xa and Xb have a bulge loop. Also, when n and m are the same integers, a stem structure formed by Xa and Xb may have a bulge loop, but from the viewpoint of stability of the MS2CP-binding motif, a stem structure having no bulge loop is preferred.

In a preferable embodiment, the OFF switch of the present invention containing two MS2CP-binding motifs contains a structure in which n and m in the Formula (II) are each independently 0 to 5 (for example, 1), and more specifically, a structure in which n and m in the Formula (II) are 1, and Xa and Xb are respectively C and G. Alternatively, it contains a structure in which n and m in the Formula (II) are each independently 5 to 10 (for example, 7), and a more specific structure can be a structure in which n and m in the Formula (II) are 7, and Xa and Xb are respectively ACGAGCG and CGCUCGU, although this is not limiting.

When the RNA switch of the present invention contains two or more binding motifs, at least two structures represented by the structure (II) may be linked to each other via a multi-branched loop. The number of nucleotides of the multi-branched loop is not also especially limited, and is, for example, 4 to 10, or 5 to 9 (for example, 7). The multi-branched loop may be linked to another structure, for example, another multi-branched loop (which multi-branched loop may further have another structure such as a helix loop structure), and for example, may be linked to 0 to 10 (for example, 2 to 5) stem structures. Herein, an integral structure containing a binding motif such as an MS2CP-binding motif, and constituted by a plurality of secondary structures, such as a structure having a plurality of structures represented by the Formula (II), is sometimes referred to as the "binding domain to the RNA-binding protein" (or simply abbreviated as "binding domain"). Such a binding domain is typically formed by at least two structures selected from a hairpin structure, a helix structure, a bulge loop, an internal loop (inteRNAl-loop), a multi-branched loop, and a pseudoknot structure.

Even when the binding motif is not the MS2CP-binding motif, corresponding binding motif and RNA domain can be designed by those skilled in the art in accordance with a corresponding RNA-binding protein. A binding motif as an L7Ae-binding motif can be appropriately designed in consideration of a known literature (such as Shi X, et al., Nat Chem Biol. 12(3): 146-152 (2016)), and a specific example includes one having a structure represented by the following Formula (III). A binding motif as an LIN28A-binding motif can be appropriately designed in consideration of known literature (such as Kawasaki S., et al., Nucleic Acids Res. 45(12): e117 (2017)), and a specific example includes one having a stem loop structure containing a GGGAU sequence. In addition, the extent of translational activation or repression can be adjusted by appropriately designing the position and sequence in the RNA, the structure, modification of a nucleotide residue and the like of the binding motif. 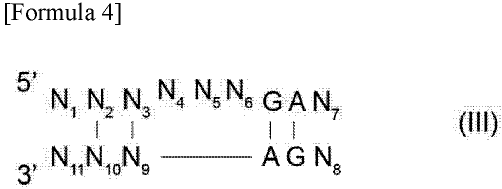 wherein
N₁ to N₁₁ each independently represent A, C, G, or U; and
N₂ and N₁₀, and N₃ and N₉ each form a base pair, or wobble base pair.

The length of the binding motif may be an optional number of bases, can be usually about 200 nucleotides or less, and is, for example, about 100 nucleotides or less, preferably about 50 nucleotides or less, and more preferably about 40 nucleotides or less. The length of the binding domain may be also an optional number of bases, can be usually about 300 nucleotides or less, and is, for example, about 200 nucleotides or less, and preferably about 150 nucleotides or less. When the total number of nucleotides is less, mass production is easier, and in addition, a large cost advantage is obtained. The lower limit of the lengths of the binding motif and the binding domain is not especially limited as long as a binding motif sequence (such as a sequence shown in SEQ ID NO: 1) is contained, and is, for example, 15 nucleotides or more, preferably 20 nucleotides or more, and more preferably 25 nucleotides or more.

The first protein of interest may be a protein to be regulated in translation, and the type of protein is not especially limited. For example, the protein of interest may be a nuclease, or may be a Cas protein or a modified product thereof. In another embodiment, the protein of interest may be an anti-CRISPR that inactivates a Cas protein. In another embodiment, the protein of interest may be a marker protein. A marker protein is a protein that is expressed from an mRNA switch, functions as a marker in a cell, and can recognize the cell. The marker protein may be, for example, a protein capable of visualization and quantitative determination by fluorescence emission, light emission, or coloration, or by assisting fluorescence emission, light emission, or coloration. Other examples of the marker protein include proteins directly affecting the function of a cell. Examples include, but are not limited to, a cell growth protein, a cell death protein, a cell signaling factor, a drug resistance gene, a transcriptional regulator, a translational regulator, a differentiation regulator, a reprogramming inducer, an RNA-binding protein factor, a chromatin regulator, and a membrane protein. For example, a cell growth protein grows only in a cell that has expressed the protein, and functions as a marker by specifying the grown cell. A cell death protein causes cell death of a cell that has expressed the protein, and kills the cell itself to function as a marker for cell life or death. As for a cell signaling factor, a cell that has expressed the factor emits a specific biological signal, and the cell signaling factor functions as a marker by specifying the signal. Examples of the cell death protein include Bax and Bim.

The OFF switch mRNA may preferably have a structure in which [5' UTR having a cap structure or a cap analog at the 5'-end, and containing a binding motif], [open reading frame containing a nucleic acid sequence encoding the first protein of interest], and [3' UTR containing a poly-A] are linked in the stated order in the 5'-end to 3'-end direction.

The cap structure or the cap analog can be selected from choices similar to those for the first mRNA. The binding motif may be present at any position as long as it is on the 3' side of the cap structure or the cap analog, and on the upstream side of the translation start codon (namely, 5' UTR). Since the translational activity of the OFF switch of the present invention can be changed depending on the position of the binding motif in the 5' UTR, the position can be appropriately adjusted in accordance with the purpose.

A spacer may be optionally contained between the cap structure or the cap analog and the binding motif, and/or between the binding motif and the start codon. The nucleic acid sequence of the spacer is not especially limited, and may be any sequence that does not form a secondary structure within the switch mRNA, and does not interact with the first and second mRNAs. In addition, the length of each spacer may be an optional number of bases, and for example, can be 0 to 800 bases, 0 to 700 bases, 0 to 600 bases, 0 to 500 bases, 0 to 450 bases, 0 to 400 bases, 0 to 350 bases, 0 to 300 bases, 0 to 250 bases, 0 to 200 bases, 0 to 150 bases, 0 to 100 bases, 0 to 50 bases, 0 to 40 bases, 0 to 30 bases, 0 to 20 bases, or 0 to 10 bases. Also, a nucleotide sequence of a restriction enzyme site or the like is included in the nucleotide sequence of the spacer. The translational activity can be adjusted by adjusting the number of bases of the spacer.

A specific example of the 5' UTR of the OFF switch mRNA includes a sequence shown in SEQ ID NO: 12 (AGGUCAGAUCCGCUAGCGGAUCCGGGAGCAGGUGAGGAUCACCCAUCUGCCAC GAGCGAGGUGAGGAUCACCCAUCUCGCUCGUGUUCCCACCGGUCGCCACC), but the sequence is not limited to a specific sequence. The 3' UTR of the OFF switch mRNA containing a poly-A can be designed in the same manner as the 3' UTRs of the first and second mRNAs.

The OFF switch mRNA functions to repress the translation of the first protein of interest by the RNA-binding protein specifically recognizing and binding to the binding motif thereof. The followings have been observed in conventional studies made by the present inventors: When the RNA-binding protein is MS2CP, as binding force between the MS2CP and the binding motif is larger, the translational activity is lower; and when a motif having two MS2CP-binding motifs and having a specific scaffold structure to strongly bind to the MS2CPs is used, the translational activity is repressed. Without being bound by any theory, it is presumed, based on these observations, that MS2CP also has a function to repress translation by binding to a binding motif, that the translational level is determined by balance between activation via the VPg protein and repression via the MS2CP, and that as the bond between the RNA and the MS2CP is stronger, the repression function via the MS2CP is increased. When two or more MS2CPs bind to the OFF switch of the present invention having a structure in which, for example, two MS2CP-binding motifs are linked to each other via a scaffold structure, the translational regulator of the present invention is in a state of always binding to the RNA. Therefore, ribosome recruited by the VPg protein is inhibited from proceeding on the RNA, resulting in probably repressing the translation of the protein encoded by the OFF switch of the present invention. Actually, also when a translational regulator containing L7Ae and a VPg protein is used instead of the translational regulator containing the MS2CP and the VPg protein, the translational repression effect is obtained by using an RNA having a motif strongly binding to L7Ae. Accordingly, in the OFF switch of the present invention, it is presumed that the protein translational repression function is caused without being limited to a specific structure when the binding property to the RNA-binding protein is increased to a prescribed or higher level by containing two or more binding motifs, with at least two or these linked via a scaffold structure, by changing the sequence of the binding motif, or by providing specific modification to a nucleotide residue.

### (B) ON Switch mRNA

The ON switch mRNA contains a binding motif that does not have a 7-methylguanosine 5'-phosphate structure at the 5'-end, and specifically recognizes an RNA-binding protein constituting the translational regulator, and a nucleic acid sequence encoding a second protein of interest.

A usual cap structure has the effect of repressing RNA degradation, and a translational activation. The ON switch mRNA preferably contains a modification at the 5' end that has only one of these effects, inhibiting RNA degradation. In other words, it may have a structure not recognized by a translational initiation factor of eIF4E. More specifically, it preferably has a structure not having a 7-methylguanosine 5'-phosphate structure at the 5'-end. An example of the modification at the 5'-end of the ON switch mRNA includes A-Cap (G(5')ppp(5')A).

Typically, the ON switch mRNA is not especially limited as long as it contains one or more binding-motifs in the 5' UTR, and the binding motif specifically recognizes and binds to an RNA-binding protein. Due to the bond between the RNA-binding protein and the RNA via the binding motif, the translation of a protein encoded by the RNA is activated. For example, when the RNA-binding protein is MS2CP, an example of the binding motif includes one having the structure represented by the Formula (II).

The second protein of interest is a protein in which translation amount is increased through the translational activation of the ON switch mRNA. The second protein of interest can be selected from the same choices described above as the examples of the first protein of interest. In a method for regulating protein translation, when the ON switch mRNA and the OFF switch mRNA are used together, the first protein of interest and the second protein of interest can be appropriately selected in accordance with a desired action. The first protein of interest and the second protein of interest can be a combination of a protein having positive activity and a protein having negative activity regarding the activity of interest, such as apoptosis activity, or nuclease activity. Examples include, but are not limited to, a combination of an apoptosis induction protein and an apoptosis repressor protein, and a combination of a Cas protein and an anti-CRISPR for inactivating a Cas protein.

The ON switch mRNA may preferably have a structure in which [5' UTR not having a 7-methylguanosine 5'-phosphate structure, and containing a binding motif], [open reading frame containing a nucleic acid sequence encoding the second protein of interest], and [3' UTR containing a poly-A] are linked in the stated order in the 5'-end to 3'-end direction.

In the 5' UTR of the ON switch mRNA, the binding motif may be provided on the 3' side of a modification structure at the 5'-end. The position of the binding motif in the 5' UTR, and the sequence and the number of spacers can be the same as those in the OFF switch mRNA.

A specific example of the 5' UTR of the ON switch mRNA includes a sequence shown in SEQ ID NO: 13 (GGGCGAAUUAAGAGAGAAAAGAAGAGUACAUGAGGAUUACCCAUGUAAGAAG AAAUAUAAGACACCGGUCGCCACC), but it is not limited to a specific sequence. The 3' UTR of the ON switch mRNA containing a poly-A can be designed in the same manner as the 3' UTRs of the first and second mRNAs.

In each of the first mRNA, second mRNA, and switch mRNA described so far, a sugar residue (ribose) of each nucleotide may be modified for purposes of, for example, reducing cytotoxicity. A site modified in the sugar residue can be, for example, a hydroxy group or hydrogen atom at position 2', position 3', and/or position 4' of the sugar residue substituted with another atom. Examples of the type of the modification include fluorination, alkoxylation (such as methoxylation or ethoxylation), O-allylation, S-alkylation (such as S-methylation or S-ethylation), S-allylation, and amination (such as -NH₂). Such modification of a sugar residue can be performed by a method known per se (see, for example, Sproat, et al., (1991), Nucle. Acid. Res. 19, 733-738; Cotton, et al., (1991), Nucl. Acid. Res. 19, 2629-2635; Hobbs, et al., (1973) Biochemistry 12, 5138-5145).

Alternatively, each sugar residue of the first, second, and switch mRNAs can be a BNA: bridged nucleic acid (LNA: linked nucleic acid) having a crosslinking structure at positions 2' and 4'. Such modification of a sugar residue can be performed also by a method known per se (see, for example, Tetrahedron Lett., 38, 8735-8738 (1997); Tetrahedron, 59, 5123-5128 (2003), Rahman S.M.A., Seki S., Obika S., Yoshikawa H., Miyashita K., Imanishi T., J. Am. Chem. Soc., 130, 4886-4896 (2008)). In addition, each of the first mRNA, second mRNA, and switch mRNAs may have a nucleobase (such as purine, or pyrimidine) modified (for example, chemically substituted). Examples of such modification includes pyrimidine modification at position 5, purine modification at position 6 and/or 8, modification with exocyclic amine, substitution with 4-thiouridine, and substitution with 5-bromo- or 5-iodo-uracil. In addition, for purposes of reducing cytotoxicity, a modified base such as pseudouridine (Ψ), N1-methylpseudouridine (N1mΨ), or 5-methylcytidine (5mC) may be contained instead of usual uridine or cytidine. The modified base can be used independently in all or some of the positions for both uridine and cytidine, and when it is used in some of the positions, the positions can be random positions at an optional rate.

In order to increase resistance to nuclease and hydrolysis, and the like, a phosphate group (such as a phosphate residue at the end) contained in the first, second, and switch mRNAs of the present invention may be modified. For example, a phosphate group of P(O)O group may be substituted with P(O)S (thioate), P(S)S (dithioate), P(O)NR₂ (amidate), P(O)R, R(O)OR', CO or CH₂ (formacetal), or 3'-amine (-NH-CH₂-CH₂-) [wherein each R or R' is independently H, or substituted or unsubstituted alkyl (such as methyl or ethyl)]. Examples of a linking group include -O-, -N-, and -S-, and the phosphate group can bind to an adjacent nucleotide via such a linking group.

The present inventors have confirmed that the binding force between an RNA-binding protein and a binding motif for the protein varies depending on the type of modification of the RNA. Accordingly, the binding force between an RNA-binding protein and a binding motif for the protein can be adjusted by appropriately changing the type of modification. Specific examples of the modification of the nucleobase, the sugar residue, and the phosphate residue described above include, but are not limited to, the following:

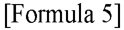

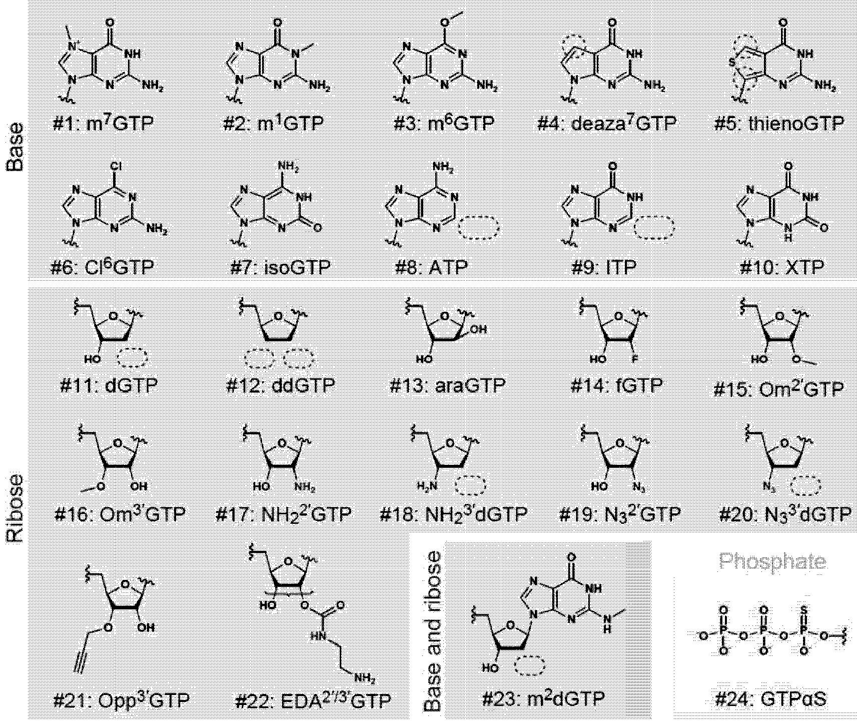

When determined in the molecular structures and the nucleic acid sequences as described above, the first mRNA, second mRNA, and switch mRNA can be synthesized by those skilled in the art in accordance with freely selected known genetic engineering methods. For example, these mRNAs can be obtained as synthetic mRNA molecules by an *in vitro* transcription method using a template DNA containing a promoter sequence as a template.

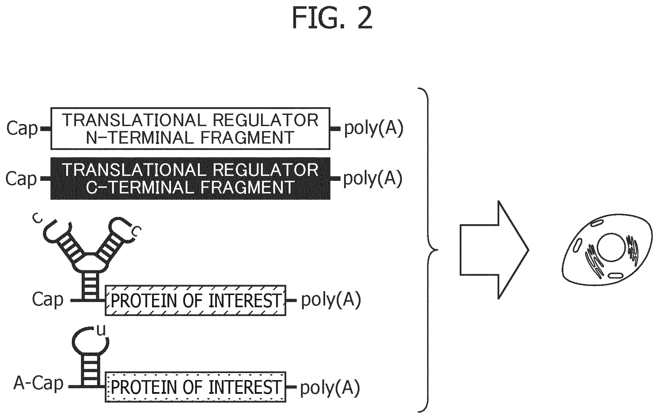

Next, introduction of the first mRNA, second mRNA, and switch mRNA into a cell will be described. In the method for regulating protein translation according to the present embodiment, as the switch mRNA, only an ON switch mRNA may be introduced, only an OFF switch mRNA may be introduced, or both may be introduced. In accordance with the intended translation regulation action, mRNA(s) to be introduced can be appropriately selected. In addition, the design of the translational regulator can be accordingly changed. FIG. 2 is a diagram schematically illustrating the introduction of the first, second, OFF switch, and ON switch mRNAs into a cell. The introduction of the mRNAs into a cell can be performed *in vitro,* or can be performed *in vivo,* which is not especially limited. For introducing the first, second, and switch mRNAs into a cell *in vitro,* any freely selected method generally employed as a method for introducing an RNA into a cell *in vitro* can be employed. As a method for directly introducing an RNA molecule into a cell, for example, the RNA molecule can be directly introduced into a cell by employing an introduction method such as a lipofection method, a polymer method, an electroporation method, a calcium phosphate coprecipitation method, a DEAE-dextran method, a microinjection method, or a gene gun method. For the introduction of the first, second, and switch mRNAs into a cell *in vivo,* a freely selected method generally employed as a method for introducing an RNA into a cell *in vivo* can be employed. For example, in a mammal, an RNA molecule can be directly introduced into a cell by employing an introduction method such as intramuscular injection, subcutaneous injection, intravenous injection, or intraarticular injection. As an advantage of the introduction of a synthetic RNA molecule, the introduced mRNA is decomposed with a half-life within about several days, and hence, it is not integrated into the genome, and therefore, the cell obtained after the introduction is easily used in medical applications and the like. In addition, a synthetic mRNA has a high transfection efficiency, and hence, is advantageous in that the switch mRNAs for expressing, in the cell, prescribed amounts of the first and second fusion proteins, and the protein of interest can be highly efficiently introduced.

The first, second, and switch mRNAs are preferably co-introduced into a cell.
Since an activity ratio between proteins expressed from two or more co-introduced mRNAs is a prescribed ratio within the cell, the first fusion protein, the second fusion protein, and the switch mRNA can be thus introduced at a prescribed ratio.

For the introduction of the first, second, and switch mRNAs into a cell, a DNA construct such as an RNA expression vector can be used. In this case, expression vectors respectively encoding the first, second, and switch mRNAs are separately designed, and two types of expression vectors can be directly introduced into a cell by an introduction method similar to that described above. As the expression vectors encoding the first mRNA and the second mRNA, those known and usually used in this field can be used, and examples include a viral vector, an artificial chromosome vector, a plasmid vector, and an expression system using transposon (sometimes designated as a transposon vector). Examples of the viral vector include a retroviral vector, a lentiviral vector, adenoviral vector, an adeno-associated viral vector, and Sendai viral vector. Examples of the artificial chromosome vector include a human artificial chromosome (HAC), a yeast artificial chromosome (YAC), and a bacterial artificial chromosome (BAC, PAC). As the plasmid vector, plasmids for mammals in general can be used, and may be, for example, an episomal vector. An example of the transposon vector includes an expression vector using a piggy Bac transposon. A DNA construct that expresses two or more of the first mRNA, the second mRNA, and the switch mRNA from a single RNA expression vector as different mRNAs can be designed.

When DNA constructs encoding the first mRNA, the second mRNA, and the switch mRNA are introduced into a cell, the first mRNA, the second mRNA, and the switch mRNA, which are transcribed within the cell from the expression vectors and generated through intracellular transcription can be caused to function similarly to directly introduced synthetic mRNAs, and thus, the first fusion protein and the second fusion protein can be expressed. The method in which a DNA construct is introduced into a cell is advantageous, for example, in a case in which these mRNAs are integrated into the genome of the cell to prepare cells stably expressing the first fusion protein, the second fusion protein, and the switch mRNA, and is advantageous in selecting or discriminating one differentiated into a specific cell type after being precedently integrated into a pluripotent stem cell or a transgenic animal.

The amounts of the first, second, and switch mRNAs introduced into a cell differ depending on the type of the target cell, and the structures of the mRNAs, and are not limited to specific amounts. An introduction amount capable of achieving the translational regulation of a desired protein of interest in the target cell can be determined by a preliminary experiment or the like.

The first mRNA and the second mRNA introduced into a cell generate the first fusion protein and the second fusion protein within the cell (FIG. 1A). Then, when the target protein is present in the cell, the first target-binding molecule and the second target-binding molecule respectively specifically recognize and bind to the first site and the second site of the target protein. Thus, a complex in which the N-terminal domain of the caged intein of the first fusion protein and the C-terminal domain of the caged intein of the second fusion protein are bound to each other is formed (FIG. 1B). Subsequently, a complex containing the target protein, the first target-binding molecule, the second target-binding molecule, and the bound caged intein is detached from the complex of FIG. 1B, thereby reconstituting the translational regulator (FIG. 1C). The reconstituted translational regulator acts on the switch mRNA for performing the translational regulation. On the other hand, when the target protein is not present in the cell, the first fusion protein and the second fusion protein expressed from the first mRNA and the second mRNA are not bound to each other, but are present in the cell as different proteins. In other words, the translational regulator is not reconstituted. When the translational regulator is not reconstituted, the first fusion protein and the second fusion protein do not perform the translational regulation of the switch mRNA, and the expression of the protein of interest encoded by the switch mRNA is not regulated.

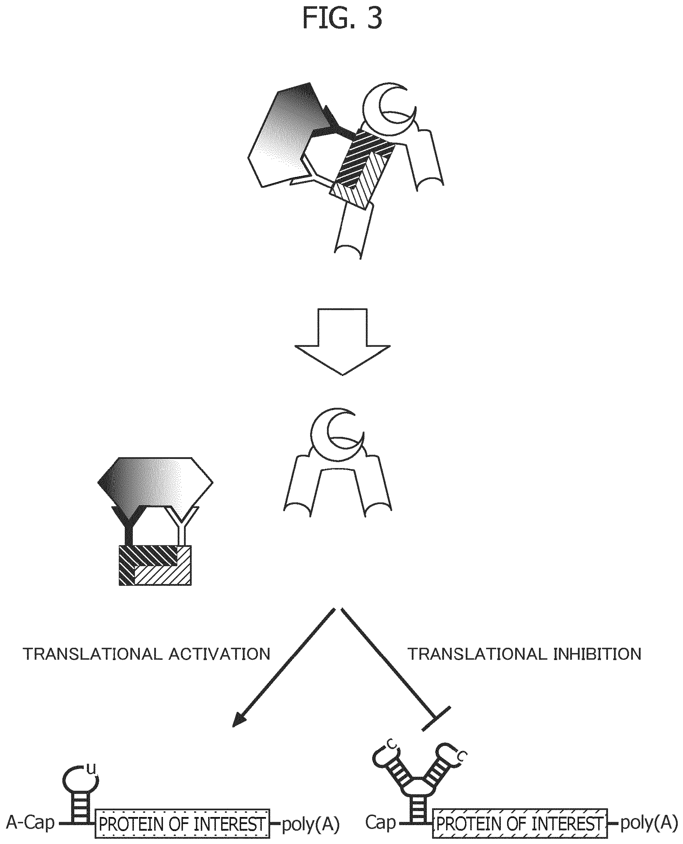

In one embodiment, the translation regulation method includes a step of introducing at least four mRNAs, that is, the first mRNA, the second mRNA, the OFF switch mRNA, and the ON switch mRNA into a cell (FIG. 2). Referring to FIG. 3, a complex of the first and second fusion proteins expressed by the first and second mRNAs is generated to reconstitute the translational regulator. The translational regulator specifically recognizes and binds to the ON switch mRNA and the OFF switch mRNA through the RNA-binding proteins. Thus, the translation of the protein of interest encoded by the ON switch mRNA can be activated, and the translation of the protein of interest encoded by the OFF switch mRNA can be repressed.

The system for regulating protein translation according to the present invention can be provided in the form of a translation regulation kit including a first mRNA, a second mRNA, and a switch mRNA, or DNAs encoding these RNAs. In this case, the translation regulation kit may include a medium suitable for culturing cells including a target cell, and instructions for dealing with the first mRNA, the second mRNA, and the switch mRNA, or the DNAs encoding these mRNAs. The instructions may include information on an experimental method for determining a suitable amount of the mRNAs to be introduced into a specific cell, and an adequate introduction amount in accordance with the cell type. According to the kit including the system for regulating protein translation according to the present embodiment, cell-selective protein translation can be regulated.

According to a second embodiment of the present invention, in the system according to the present invention, the mRNA (a) and the mRNA (b) may be a single mRNA. In the single mRNA, one mRNA molecule may be able to express the first and second fusion proteins as separate different molecules. More specifically, the single mRNA contains, in the 5' to 3' direction, a nucleic acid sequence encoding the second fusion protein, a self-cleaving sequence encoding a self-cleaving peptide, and a nucleic acid sequence encoding the first fusion protein.

Also, in the single mRNA, the 5' UTR and the 3' UTR can be designed in the same manner as those of the first or second mRNA. The single mRNA can be designed in such a manner that the open reading fame contains, in the 5'-end to 3'-end direction, a nucleic acid sequence encoding the second fusion protein, a self-cleaving sequence encoding a self-cleaving peptide, and a nucleic acid sequence encoding the first fusion protein in the stated order. The self-cleaving peptide may be a virus 2A peptide, or a 2A-like peptide having a similar function. Examples include, but are not limited to, F2A, E2A, T2A, and P2A. The form of the modification of a sugar residue and the like in the design of the mRNA molecule may be the same as the modification form described in the first embodiment.

Also, the translation regulation method according to the second embodiment includes, similar to that of the first embodiment, a step of introducing the single mRNA expressing the first and second fusion proteins and the switch mRNA into a cell, and can be performed in the same manner as that of the first embodiment. The system and method for regulating the translation according to the second embodiment have advantages that there is no need to separately prepare and introduce the first and second mRNAs, and that a possibility of production of a cell expressing only one of the first and second fusion proteins can be eliminated.

### EXAMPLES

Now, the present invention will be described in more detail with reference to examples. It is noted that the following examples do not limit the present invention.

### Experimental Methods

### Construction of pDNA

An insert was produced by PCR using PrimeSTAR Max DNA Polymerase (Takara Bio Inc.), and was purified with Monarch PCR & DNA Cleanup Kit (New England Biolabs). In addition, cloning was performed with In-Fusion HD Cloning Kit (Takara Bio Inc.). A pDNA was amplified with *E. coli* DH5α or HST08 strain, and purified with Monarch plasmid miniprep kit (New England Biolabs). For measuring a DNA concentration, Nanodrop ONE (Thermo Fisher Scientific) was used. The sequence of a constructed plasmid was confirmed by Sanger DNA sequencing service (Genewiz). The names and SEQ ID NOS of plasmids are shown in Table 2 below.

**Table 2**

| Name | Sequence ID Number |
|---|---|
| pcDNA3.1-MS2CP(1-45)-eNpuNcage-Nb113 | 16 |
| pcDNA3.1-CA1698-NpuCcage-MS2CP(46-116)-VPg(FCV) | 17 |
| pSV40-2xScMS2(C)-Luc2 | 18 |
| pUTR2-MS2CP(1-45)-eNpuNcage-Nb113 | 19 |
| pUTR2-CA169-NpuCcage-MS2CP(46-116)-VPg(FCV) | 20 |
| pUTR2-MS2CP(1-45)-eNpuNcage-Lag16 | 21 |
| pUTR2-GFPenhancerNb-NpuCcage-MS2CP(46-116)-VPg(FCV) | 22 |

### in vitro Transcription of mRNA

A template DNA was produced by PCR using PrimeSTAR Max DNA Polymerase, and purified with Monarch PCR & DNA Cleanup Kit (New England Biolabs). This template DNA was used as a template to perform a transcription reaction with MEGAscript T7 Transcription Kit (Thermo Fisher Scientific) at 37°C for about 6 hours. In this case, 6 mM ATP, CTP, GTP, and N1-methylpseudo UTP, and 4.8 mM CleanCap Reagent AG (3' Ome) (TriLink) were used as NTP and cap analog. After completing the reaction, the resultant was purified with RNACleanXP (Nippon Genetics Co., Ltd.), subjected to a dephosphorylation reaction with Quick CIP (New England Biolabs) at 37°C for 30 minutes, and then purified with RNeasy Mini Kit (Qiagen). The RNA concentration was measured with Nanodrop ONE. In addition, the size of the purified mRNA was confirmed with Bioanalyzer and RNA 6000 Nanokit (Agilent Technologies Japan, Ltd.). The names and SEQ ID NOS of template DNAs are shown in Table 3 below.

**Table 3**

| Name | Sequence ID Number |
|---|---|
| MS2CP(1-45)-eNpuNcage-Nb113 template DNA for in vitro transcription | 23 |
| CA1698-NpuCcage-MS2CP(46-116)-VPg(FCV) template DNA for in vitro transcription | 24 |
| MS2CP(1-45)-eNpuNcage-Lag16 template DNA for in vitro transcription | 25 |
| GFPenhancerNb-NpuCcage-MS2CP(46-116)-VPg(FCV) template DNA for in vitro transcription | 26 |
| eDHFR-DYKtag template DNA for in vitro transcription | 27 |
| EGFP template DNA for in vitro transcription | 28 |

### Introduction of Plasmid DNA into Cell

1 x 10⁴ HeLa cells were seeded in each well of a 96-well transparent white plastic flat bottom plate to be cultured in a CO₂ incubator at 37°C for 1 day. Thereafter, the pDNA was transfected into the cells of each well with 0.3 µl of TransIT-X2 (Takara Bio Inc.).

### Introduction of Messenger RNA into Cell

1 x 10⁴ HeLa cells were seeded in each well of a 96-well transparent white plastic flat bottom plate to be cultured in a CO₂ incubator at 37°C for 1 day. Thereafter, the mRNA was transfected into the cells of each well with 0.2 µl of Lipofectamine MessengerMAX (Thermo Fisher Scientific).

### Luciferase Assay

After culturing the cells in a CO₂ incubator at 37°C for 1 to 2 days after the transfection, Nano-Glo Dual-Luciferase Reporter Assay System (Promega) and GloMax Navigator Microplate Luminometer (Promega) were respectively used to measure luciferase activity of Luc2 and NanoLuc. Luc2 emission was corrected with emission of NanoLuc used as a transfection control, and was expressed as an absolute value calculated assuming that a negative control group of each experiment had a value of 1.

### Example 1: Translational Repression Induced by Detection of eDHFR in Introduction of Plasmid DNA

In order to repress or activate the translation of a switch mRNA only in the presence of a target protein, a translational regulator was split in an RNA-binding protein, and a caged intein and a nanobody corresponding to a target-binding molecule (an antigen-binding domain of a single chain antibody) were fused with each of the split fragments. As the translational regulator to be split, Caliciviral VPg-based Translational activator (CaVT, SEQ ID NO: 11) that can be used for both translational repression and translational activation by changing the sequence of a switch mRNA to be regulated was selected. When the target protein is present, both the fragments of the split CaVT bind to the target protein via the nanobodies. Thus, a full length CaVT is reconstituted owing to the action of the caged intein to bind to the switch mRNA in this method (FIGS. 1 and 2).

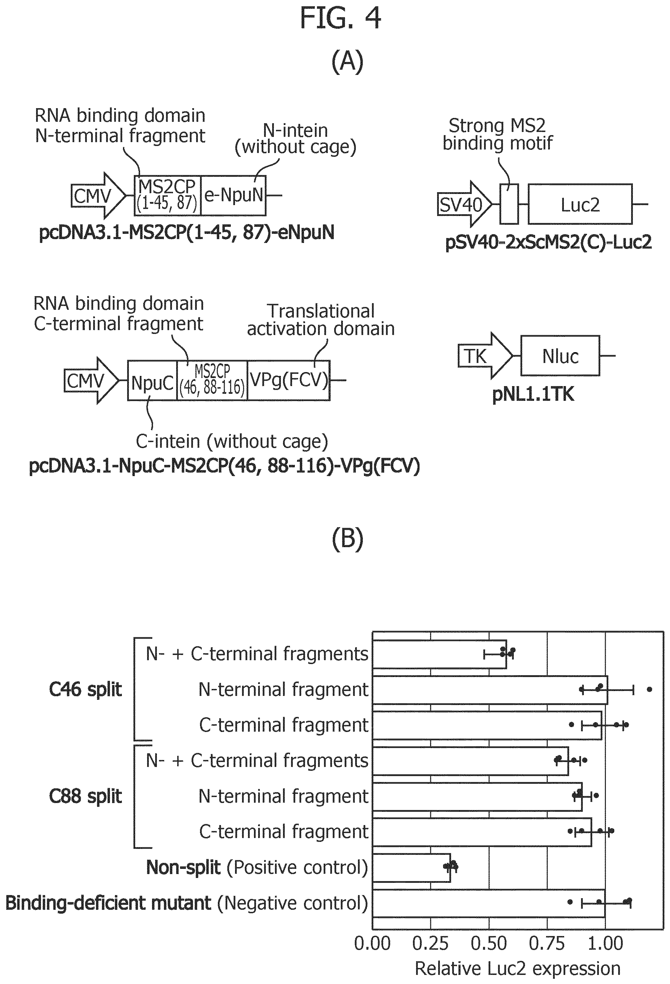

First, it was examined in which position the CaVT was to be split for enabling the reconstitution by the caged intein. The caged intein originates from *Nostoc punctiforme* (Npu)-derived DnaE intein, and for reconstitution by Npu DnaE intein, an amino acid at the N-terminal in the C-terminal side fragment needs to be cysteine. Since cysteines contained in the RNA-binding protein of the CaVT are two residues at positions 46 and 88, a plasmid DNA expressing one in which Npu DnaE intein was fused with a CaVT fragment split in either of these positions was constructed (FIG. 4A). Since the caged intein is not used, but the original Npu DnaE intein is used, in this plasmid DNA, the reconstitution of the CaVT occurs even when the target protein is not present as long as both the N-terminal side and C-terminal side CaVT fragments are present. These plasmid DNAs were introduced into the HeLa cells together with a Luc2-expressing plasmid DNA in which a strong CaVT-binding motif had been added to the 5' non-coding region to be repressed in translation by the CaVT. In using one obtained by splitting the CaVT in the residue at position 46, when both the N-terminal side and C-terminal side CaVT fragments were expressed, the translational repression of Luc2 was observed. On the other hand, in using one obtained by splitting the CaVT in the residue at position 88, definite translational repression was not observed (FIG. 4B).

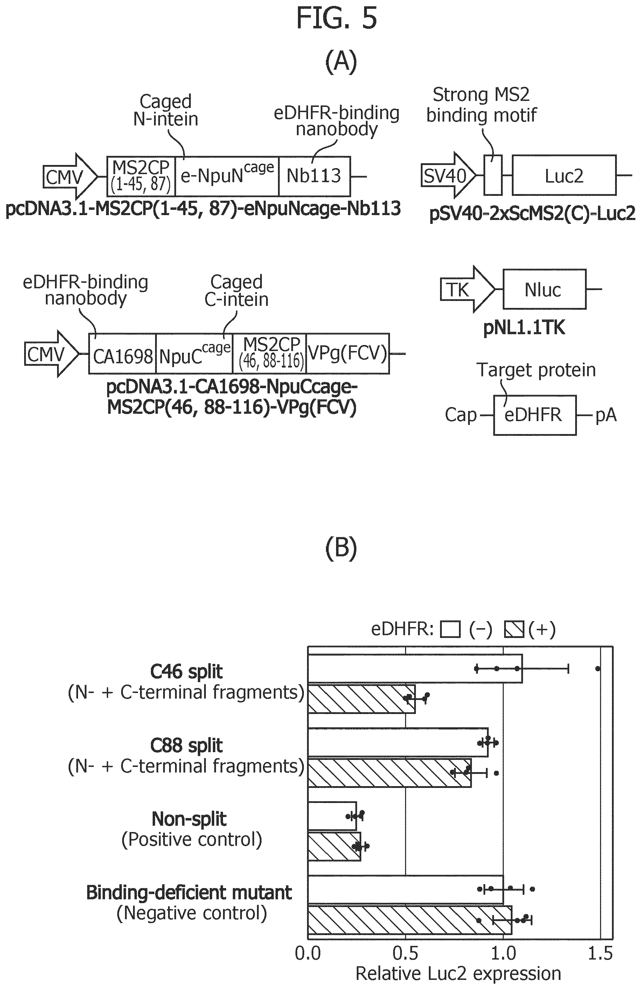

Next, in order to examine the CaVT reconstitution by a target protein and the translational repression caused thereby, an expression plasmid DNA of one obtained by fusing a caged intein and a nanobody with each of the CaVT fragments split respectively in the residues at positions 46 and 88 was constructed (FIG. 5A). Here, as the nanobody, Nb113 and CA1698 that are nanobodies binding to *E*. *coli*-derived dihydrofolate reductase (eDHFR) were respectively used. In the same manner as in FIG. 2, these plasmid DNAs were introduced into the HeLa cells together with a Luc2-expressing plasmid DNA designed to be repressed in translation by the CaVT. In this case, an mRNA expressing eDHFR was simultaneously introduced. As a result, the translational repression was induced by eDHFR in one obtained by splitting the CaVT in the residue at position 46. On the other hand, the translational repression by eDHFR was not induced in one obtained by splitting the CaVT in the residue at position 88 (FIG. 5B).

### Example 2: Translational Repression and Translational Activation Induced by Detection of eDHFR in Introduction of mRNA

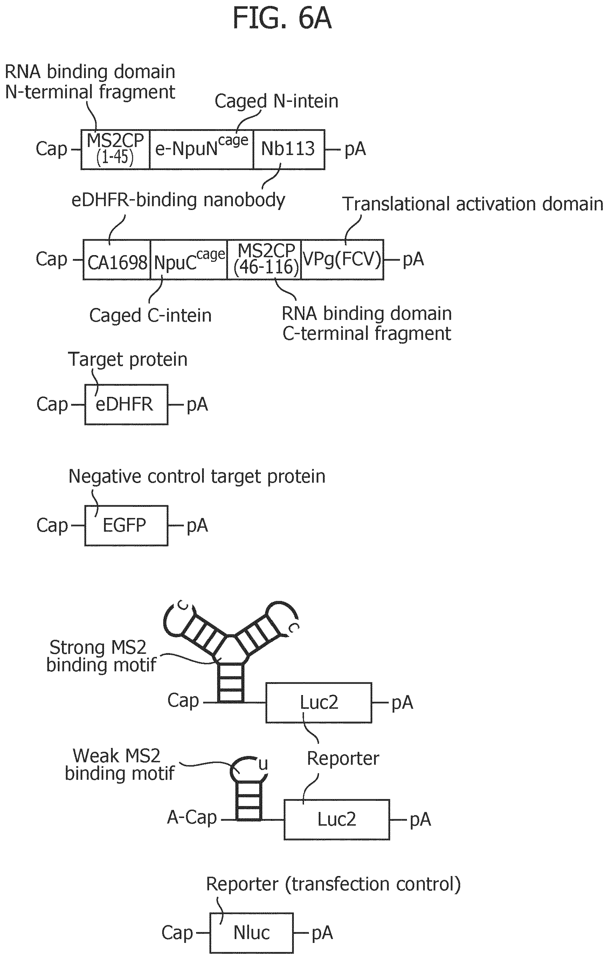

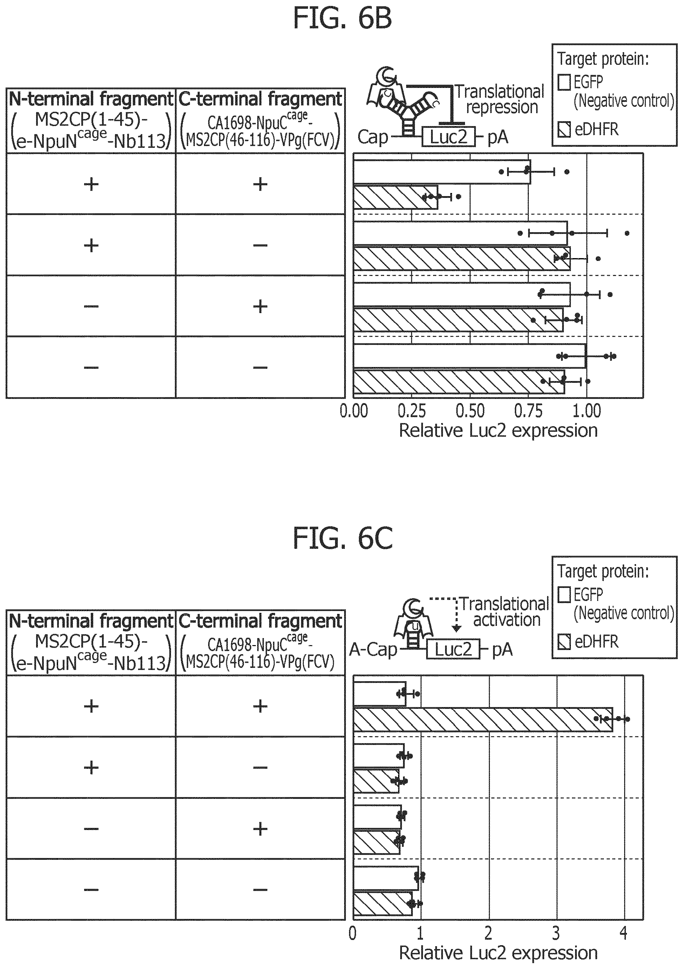

Since the translational repression by eDHFR detection could be confirmed in the plasmid DNA, it was next decided to examine whether or not the translational repression could be caused by eDHFR detection also when a split CaVT or a translation regulation target thereof was introduced with an mRNA. An mRNA expressing the split CaVT having the same configuration as that of FIG. 3, an mRNA expressing eDHFR, and a Luc2 mRNA in which a strong CaVT-binding motif had been added to the 5' non-coding region to be repressed in translation by the CaVT were all introduced into the HeLa cells. Only when both the N-terminal side and C-terminal side fragments of the split CaVT were introduced was the translational repression of Luc2 induced in response to eDHFR (FIGS. 6A and 6B).

Next, it was examined whether or not translational activation could be also induced in response to eDHFR by using this split CaVT. It has been already reported that an mRNA having a weak CaVT-binding motif in the 5' non-coding region, and using A-Cap that is a translationally inactive analog as the 5'-cap structure is activated in translation by CaVT (Non-Patent Document 1). A Luc2 mRNA having a structure necessary for the translational activation by CaVT was produced, and was introduced into the HeLa cell together with a split CaVT mRNA and an eDHFR mRNA. Only when both the N-terminal side and C-terminal side fragments of the split CaVT were present was translational activation in response to eDHFR observed (FIGS. 6A and 6C).

### Example 3: Translational Repression and Translational Activation Induced by EGFP Detection in Introduction of mRNA

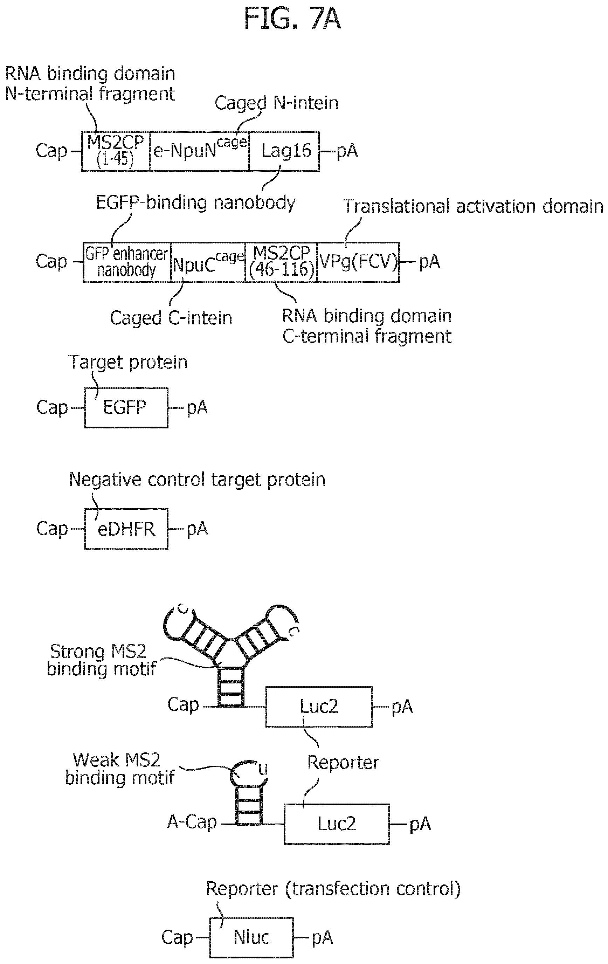

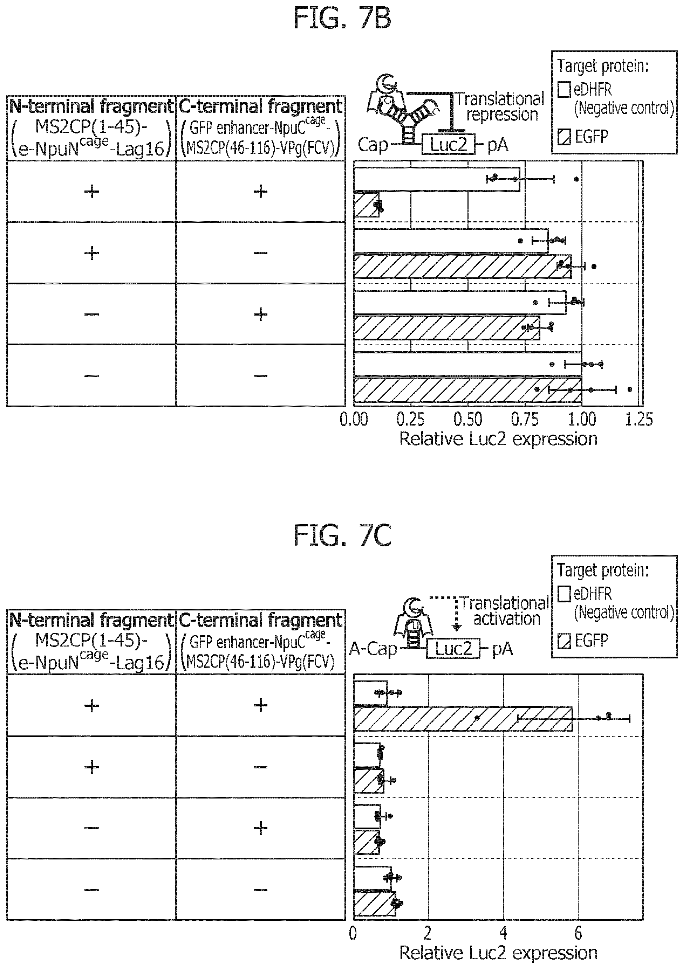

In order to show the versatility of the present system capable of inducing translational repression or translational activation by detecting any protein by changing the site of a nanobody binding to a target protein, a split CaVT in which nanobody portions thereof was changed from Nb113 and CA1698 binding to eDHFR to Lag16 and GFP enhancer Nb binding to EGFP was constructed. When an mRNA of this split CaVT designed for EGFP detection was introduced into the HeLa cells together with an EGFP mRNA and a Luc2 mRNA having a strong CaVT-binding motif was the translational repression of Luc2 induced by the target protein in the same manner as in Example 2 (FIGS. 7A and 7B). In addition, also when a Luc2 mRNA having a weak CaVT-binding motif and A-Cap was used was translational activation induced target protein-dependently in the same manner as in Example 2 (FIGS. 7A and 7C). It is believed that these results show the versatility of the present system.

## Claims

1. A system for regulating protein translation, comprising the following mRNAs (a), (b), and (c), or DNAs encoding these mRNAs:
(a) an mRNA that expresses a first fusion protein containing a first target-binding molecule specifically recognizing a first site of a target molecule, an N-terminal domain of a caged intein, and an N-terminal domain of a translational regulator;
(b) an mRNA that expresses a second fusion protein containing a second target-binding molecule specifically recognizing a second site of the target molecule, a C-terminal domain of a caged intein, and a C-terminal domain of the translational regulator; and
(c) a switch mRNA containing one or more binding motifs specifically recognizing the translational regulator, and a nucleic acid sequence encoding a protein of interest,
wherein the translational regulator contains an RNA-binding protein.

2. The system according to claim 1, wherein the mRNA (a) is a first mRNA containing a nucleic acid sequence encoding the first fusion protein, and the mRNA (b) is a second mRNA containing a nucleic acid sequence encoding the second fusion protein.

3. The system for regulating protein translation according to claim 1, wherein the mRNA (a) and the mRNA (b) are a single mRNA containing, in a 5' to 3' direction, a nucleic acid sequence encoding the second fusion protein, a self-cleaving sequence encoding a self-cleaving peptide, and a nucleic acid sequence encoding the first fusion protein.

4. The system according to any one of claims 1 to 3, wherein the translational regulator contains the RNA-binding protein and a translational activator.

5. The system according to any one of claims 1 to 4, wherein the N-terminal domain of the translational regulator contains a first portion of the RNA-binding protein, and the C-terminal domain of the translational regulator contains a second portion of the RNA-binding protein, and a translational activator.

6. The system according to any one of claims 1 to 5, wherein the N-terminal domain of the caged intein is a caged eNpu N-intein or a mutant thereof, and the C-terminal domain of the caged intein is a caged Npu C-intein or a mutant thereof.

7. The system according to any one of claims 1 to 6, wherein the switch mRNA is:
(A) an OFF switch mRNA repressed in translation by the translational regulator, and/or
(B) an ON switch mRNA activated in translation by the translational regulator,
the OFF switch mRNA has a cap structure or a cap analog at a 5'-end, and contains one or more binding motifs specifically recognizing the RNA-binding protein, and a nucleic acid sequence encoding a first protein of interest, and
the ON switch mRNA does not have a 7-methylguanosine 5'-phosphate structure, and contains one or more binding motifs specifically recognizing the RNA-binding protein, and a nucleic acid sequence encoding a second protein of interest.

8. The system according to any one of claims 1 to 7, wherein the RNA-binding protein is MS2CP.

9. The system according to claim 8, wherein a binding motif for the MS2CP has a potential secondary structure represented by the following Formula (I): 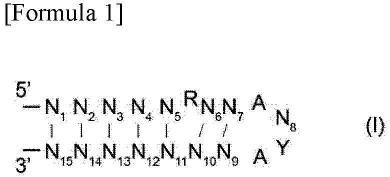 wherein
N₁ to N₁₅ each independently represent A, C, G, or U;
R represents G or A;
Y represents U or C; and
N₁ and N₁₅, N₂ and N₁₄, N₃ and N₁₃, N₄ and N₁₂, N₅ and N₁₁, N₆ and N₁₀, and N₇ and N₉ each form a base pair or wobble base pair.

10. The system according to claim 8 or 9, wherein the OFF switch mRNA contains two or more binding motifs specifically recognizing the MS2CP, and at least two of the two or more binding motifs are linked to each other via an RNA scaffold structure.

11. A method for regulating protein translation, comprising a step of introducing the system according to any one of claims 1 to 10 into a cell in vitro.

12. The system according to any one of claims 1 to 10 for use in a method of regulating protein translation, the method comprising a step of introducing the system into a cell in vivo.

13. The system for regulating protein translation according to claim 1,
wherein the translational regulator is a protein containing:
an amino acid sequence specified by SEQ ID NO: 11;
an amino acid sequence obtained by deleting, substituting, inserting, and/or adding one to five amino acids in the amino acid sequence specified by SEQ ID NO: 11; or
an amino acid sequence having a sequence identity of 85% or more to the amino acid sequence shown in SEQ ID NO: 11, and
a splitting portion between an N-terminal domain and a C-terminal domain of the translational regulator is between a residue at position 45 and a residue at position 46, or a portion away from the splitting portion toward an N-terminal side or a C-terminal side by five amino acid residues or less.

14. The system for regulating protein translation according to claim 1, wherein the translational regulator contains an amino acid sequence specified by SEQ ID NO: 11, the N-terminal domain of the translational regulator contains residues at positions 1 to 45 of SEQ ID NO: 11, and the C-terminal domain of the translational regulator contains residues at positions 46 to 116 of SEQ ID NO: 11.

## Patentansprüche

1. System zur Regulierung der Proteintranslation, umfassend die folgenden mRNAs (a), (b) und (c) oder DNAs, die für diese mRNAs kodieren:
(a) eine mRNA, die ein erstes Fusionsprotein exprimiert, das ein erstes zielbindendes Molekül enthält, das spezifisch eine erste Stelle eines Zielmoleküls, eine N-terminale Domäne eines Caged-Inteins und eine N-terminale Domäne eines translationalen Regulators erkennt;
(b) eine mRNA, die ein zweites Fusionsprotein exprimiert, das ein zweites zielbindendes Molekül enthält, das spezifisch eine zweite Stelle des Zielmoleküls, eine C-terminale Domäne eines Caged-Inteins und eine C-terminale Domäne des translationalen Regulators erkennt; und
(c) eine Switch-mRNA, die ein oder mehrere Bindungsmotive enthält, die den translationalen Regulator spezifisch erkennen, und eine Nukleinsäuresequenz, die für ein Protein von Interesse kodiert,
wobei der translationale Regulator ein RNA-bindendes Protein enthält.

2. System nach Anspruch 1, wobei die mRNA (a) eine erste mRNA ist, die eine Nukleinsäuresequenz enthält, die für das erste Fusionsprotein kodiert, und die mRNA (b) eine zweite mRNA ist, die eine Nukleinsäuresequenz enthält, die für das zweite Fusionsprotein kodiert.

3. System zur Regulierung der Proteintranslation nach Anspruch 1, wobei die mRNA (a) und die mRNA (b) eine einzelne mRNA sind, die, in einer 5'-zu-3' -Richtung, eine Nukleinsäuresequenz, die für das zweite Fusionsprotein kodiert, eine selbstspaltende Sequenz, die für ein selbstspaltendes Peptid kodiert, und eine Nukleinsäuresequenz, die für das erste Fusionsprotein kodiert, enthält.

4. System nach einem der Ansprüche 1 bis 3, wobei der translationale Regulator das RNA-bindende Protein und einen translationalen Aktivator enthält.

5. System nach einem der Ansprüche 1 bis 4, wobei die N-terminale Domäne des translationalen Regulators einen ersten Abschnitt des RNA-bindenden Proteins enthält und die C-terminale Domäne des translationalen Regulators einen zweiten Abschnitt des RNA-bindenden Proteins und einen translationalen Aktivator enthält.

6. System nach einem der Ansprüche 1 bis 5, wobei die N-terminale Domäne des Caged-Inteins ein Caged-eNpu-N-Intein oder eine Mutante davon ist und die C-terminale Domäne des Caged-Inteins ein Caged-Npu-C-Intein oder eine Mutante davon ist.

7. System nach einem der Ansprüche 1 bis 6, wobei die Switch-mRNA Folgendes ist:
(A) eine OFF-Switch-MRNA, die von dem translationalen Regulator bei der Translation repressiert wird, und/oder
(B) eine ON-Switch-mRNA, die von dem translationalen Regulator bei der Translation aktiviert wird,
die OFF-Switch-mRNA eine Cap-Struktur oder ein Cap-Analogon an einem 5'-Ende aufweist und ein oder mehrere Bindungsmotive enthält, die spezifisch das RNA-bindende Protein erkennen, und eine Nukleinsäuresequenz, die für ein erstes Protein von Interesse kodiert, und
die ON-Switch-mRNA keine 7-Methylguanosin-5'-Phosphat-Struktur aufweist und ein oder mehrere Bindungsmotive enthält, die spezifisch das RNA-bindende Protein erkennen, und eine Nukleinsäuresequenz, die für ein zweites Protein von Interesse kodiert.

8. System nach einem der Ansprüche 1 bis 7, wobei das RNA-bindende Protein MS2CP ist.

9. System nach Anspruch 8, wobei ein Bindungsmotiv für das MS2CP eine potenzielle sekundäre Struktur aufweist, die durch die folgende Formel (I) dargestellt ist: 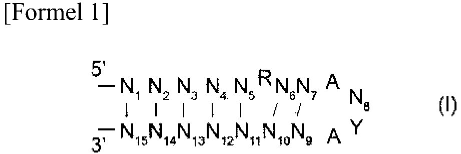 wobei gilt:
N₁ bis N₁₅ stellen jeweils unabhängig A, C, G oder U dar;
R stellt G oder A dar;
Y stellt U oder C dar; und
N₁ und N₁₅, N₂ und N₁₄, N₃ und N₁₃, N₄ und N₁₂, N₅ und N₁₁, N₆ und N₁₀ und N₇ und N₉ bilden jeweils ein Basispaar oder Wobbel-Basispaar.

10. System nach Anspruch 8 oder 9, wobei die OFF-Switch-mRNA zwei oder mehr Bindungsmotive enthält, die spezifisch das MS2CP erkennen, und mindestens zwei der zwei oder mehr Bindungsmotive über eine RNA-Gerüststruktur miteinander verknüpft sind.

11. Verfahren zur Regulierung der Proteintranslation, umfassend einen Schritt des Einführens des Systems nach einem der Ansprüche 1 bis 10 in eine Zelle in vitro.

12. System nach einem der Ansprüche 1 bis 10 zur Verwendung in einem Verfahren zum Regulieren der Proteintranslation, wobei das Verfahren einen Schritt des Einführens des Systems in eine Zelle in vivo umfasst.

13. System zur Regulierung der Proteintranslation nach Anspruch 1,
wobei der translationale Regulator ein Protein ist, das Folgendes enthält:
eine Aminosäuresequenz, die vorgegeben ist von SEQ ID NO: 11;
eine Aminosäuresequenz, die erhalten wird durch Deletieren, Substituieren, Einfügen und/oder Hinzufügen von einer bis fünf Aminosäuren in der in von SEQ ID NO: 11 vorgegebenen Aminosäuresequenz; oder
eine Aminosäuresequenz mit einer Sequenzidentität von 85 % oder mehr zu der in SEQ ID NO: 11 gezeigten Aminosäuresequenz, und
ein Spaltabschnitt zwischen einer N-terminalen Domäne und einer C-terminalen Domäne des translationalen Regulators zwischen einem Rest an Position 45 und einem Rest an Position 46 oder ein Abschnitt weg von dem Spaltabschnitt hin zu einer N-terminalen Seite oder einer C-terminalen Seite um fünf Aminosäurereste oder weniger ist.

14. System zur Regulierung der Proteintranslation nach Anspruch 1, wobei der translationale Regulator eine Aminosäuresequenz enthält, die von SEQ ID NO: 11 vorgegeben ist, die N-terminale Domäne des translationalen Reglers Reste an Position 1 bis 45 von SEQ ID NO: 11 enthält und die C-terminale Domäne des translationalen Regulators Reste an Position 46 bis 116 von SEQ ID NO: 11 enthält.

## Revendications

1. Système de régulation de la traduction de protéines, comprenant les ARNm (a), (b) et (c) suivants, ou des ADN codant pour ces ARNm :
(a) un ARNm qui exprime une première protéine de fusion contenant une première molécule de liaison à une cible reconnaissant spécifiquement un premier site d'une molécule cible, un domaine N-terminal d'une intéine cagée et un domaine N-terminal d'un régulateur de traduction ;
(b) un ARNm qui exprime une seconde protéine de fusion contenant une seconde molécule de liaison à une cible reconnaissant spécifiquement un second site de la molécule cible, un domaine C-terminal d'une intéine cagée et un domaine C-terminal du régulateur de traduction ; et
(c) un ARNm commutateur contenant un ou plusieurs motifs de liaison reconnaissant spécifiquement le régulateur de traduction, et une séquence d'acide nucléique codant pour une protéine d'intérêt,
dans lequel le régulateur de traduction contient une protéine de liaison à l'ARN.

2. Système selon la revendication 1, dans lequel l'ARNm (a) est un premier ARNm contenant une séquence d'acide nucléique codant pour la première protéine de fusion, et l'ARNm (b) est un second ARNm contenant une séquence d'acide nucléique codant pour la seconde protéine de fusion.

3. Système de régulation de la traduction de protéines selon la revendication 1, dans lequel l'ARNm (a) et l'ARNm (b) sont un ARNm unique contenant, dans une direction 5' à 3', une séquence d'acide nucléique codant pour la seconde protéine de fusion, une séquence auto-clivante codant pour un peptide auto-clivant, et une séquence d'acide nucléique codant pour la première protéine de fusion.

4. Système selon l'une quelconque des revendications 1 à 3, dans lequel le régulateur de traduction contient la protéine de liaison à l'ARN et un activateur de traduction.

5. Système selon l'une quelconque des revendications 1 à 4, dans lequel le domaine N-terminal du régulateur de traduction contient une première partie de la protéine de liaison à l'ARN, et le domaine C-terminal du régulateur de traduction contient une seconde partie de la protéine de liaison à l'ARN, et un activateur de traduction.

6. Système selon l'une quelconque des revendications 1 à 5, dans lequel le domaine N-terminal de l'intéine cagée est une N-intéine eNpu cagée ou un mutant de celle-ci, et le domaine C-terminal de l'intéine cagée est une C-intéine Npu cagée ou un mutant de celle-ci.

7. Système selon l'une quelconque des revendications 1 à 6, dans lequel l'ARNm commutateur est :
(A) un ARNm commutateur OFF dont la traduction est réprimée par le régulateur de traduction, et/ou
(B) un ARNm commutateur ON dont la traduction est activée par le régulateur de traduction,
l'ARNm commutateur OFF présente une structure de coiffe ou un analogue de coiffe à une extrémité 5', et contient un ou plusieurs motifs de liaison reconnaissant spécifiquement la protéine de liaison à l'ARN, et une séquence d'acide nucléique codant pour une première protéine d'intérêt, et
l'ARNm commutateur ON ne présente pas de structure de 7-méthylguanosine 5'-phosphate, et contient un ou plusieurs motifs de liaison reconnaissant spécifiquement la protéine de liaison à l'ARN, et une séquence d'acide nucléique codant pour une seconde protéine d'intérêt.

8. Système selon l'une quelconque des revendications 1 à 7, dans lequel la protéine de liaison à l'ARN est la MS2CP.

9. Système selon la revendication 8, dans lequel un motif de liaison pour la MS2CP présente une structure secondaire potentielle représentée par la Formule (I) suivante : 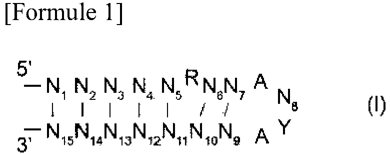 où
N₁ à N₁₅ représentent chacun indépendamment A, C, G ou U ;
R représente G ou A ;
Y représente U ou C ; et
N₁ et N₁₅, N₂ et N₁₄, N₃ et N₁₃, N₄ et N₁₂, N₅ et N₁₁, N₆ et N₁₀, et N₇ et N₉ forment chacun une paire de bases ou une paire de bases oscillante.

10. Système selon l'une des revendications 8 ou 9, dans lequel l'ARNm commutateur OFF contient deux motifs de liaison ou plus reconnaissant spécifiquement la MS2CP, et au moins deux des deux motifs de liaison ou plus sont liés l'un à l'autre par une structure d'échafaudage d'ARN.

11. Procédé permettant la régulation de la traduction de protéines, comprenant une étape d'introduction du système selon l'une quelconque des revendications 1 à 10 dans une cellule in vitro.

12. Système selon l'une quelconque des revendications 1 à 10, destiné à être utilisé dans un procédé de régulation de la traduction de protéines, le procédé comprenant une étape d'introduction du système dans une cellule in vivo.

13. Système de régulation de la traduction de protéines selon la revendication 1,
dans lequel le régulateur de traduction est une protéine contenant :
une séquence d'acides aminés spécifiée par SEQ ID N° : 11 ;
une séquence d'acides aminés obtenue par délétion, substitution, insertion et/ou ajout d'un à cinq acides aminés dans la séquence d'acides aminés spécifiée par SEQ ID N° : 11 ; ou
une séquence d'acides aminés présentant une identité de séquence de 85 % ou plus avec la séquence d'acides aminés représentée dans SEQ ID N° : 11 ; et
une partie de division située entre un domaine N-terminal et un domaine C-terminal du régulateur de traduction est située entre un résidu en position 45 et un résidu en position 46, ou une partie éloignée de la partie de division vers un côté N-terminal ou un côté C-terminal de cinq résidus d'acides aminés ou moins.

14. Système de régulation de la traduction de protéines selon la revendication 1, dans lequel le régulateur de traduction contient une séquence d'acides aminés spécifiée par SEQ ID N° : 11, le domaine N-terminal du régulateur de traduction contient des résidus aux positions 1 à 45 de SEQ ID N° : 11, et le domaine C-terminal du régulateur de traduction contient des résidus aux positions 46 à 116 de SEQ ID N° : 11.
